# EUROPEAN PATENT SPECIFICATION

(11) **EP 2 844 739 B1**
(45) Date of publication and mention of the grant of the patent: **19.06.2019**
(21) Application number: 13784648.1
(22) Date of filing: 30.04.2013
(51) Int. Cl.: C12N 5/071, C12N 5/02, C12N 5/073, C12P 21/02, A61K 35/39

(54) **METHODS AND COMPOSITIONS FOR GENERATING PANCREATIC PROGENITORS AND FUNCTIONAL BETA CELLS FROM hPSCs**
VERFAHREN UND ZUSAMMENSETZUNGEN ZUR ERZEUGUNG PANKREATISCHER VORLÄUFER UND FUNKTIONELLER BETA-ZELLEN AUS HPSCS
PROCÉDÉS ET COMPOSITIONS POUR LA GÉNÉRATION DE PROGÉNITEURS PANCRÉATIQUES ET DE CELLULES BÊTA FONCTIONNELLES À PARTIR DE HPSC

(30) Priority: 30.04.2012 US 201261640366 P
(43) Date of publication of application: 11.03.2015
(73) Proprietor: University Health Network, Toronto, Ontario M5G 1L7 (CA)
(72) Inventor: KELLER, Gordon, Toronto, Ontario M4W 2E6 (CA); NOSTRO, Maria Cristina, Toronto, Ontario M5B 1Y6 (CA); HOLTZINGER, Audrey, Toronto, Ontario M5G 2J8 (CA); SARANGI, Farida, Thornhill, Ontario L3T 7C8 (CA)
(74) Representative: Greaves Brewster LLP
(86) International application number: PCT/CA2013/000432
(87) International publication number: WO 2013/163739

(56) References cited:
- WO-A2-03/050249
- WO-A2-2012/070014
- WO-A2-2012/168930
- ZHANG, D. ET AL.: 'Highly efficient differentiation of human ES cells and iPS cells into mature pancreatic insulin-producing cells' CELL RESEARCH vol. 19, no. 4, April 2009, pages 429 - 438, XP055074920
- JIANG, J. ET AL.: '``Generation of insulin-producing islet-like clusters from human embryonic stem cells' STEM CELLS vol. 25, no. 8, August 2007, pages 1940 - 1953, XP008084029
- MICALLEF, S. ET AL.: '`INSGFP/u human embryonic stem cells facilitate isolation of in vitro derived insulin-producing cells' DIABETOLOGIA vol. 55, no. 3, March 2012, pages 694 - 706, XP035009998
- BANERJEE, I. ET AL.: 'Impact of co-culture on pancreatic differentiation of embryonic stem cells' JOURNAL OF TISSUE ENGINEERING AND REGENERATIVE MEDICINE vol. 5, no. 4, April 2011, pages 313 - 23, XP055175521
- TALAVERA-ADAME, D. ET AL.: 'Endothelial cells in co-culture enhance embryonic stem cell differentiation to pancreatic progenitors and insulin- producing cells through BMP signaling' STEM CELL REVIEWS AND REPORTS vol. 7, no. 3, September 2011, pages 532 - 543, XP019927860
- NOSTRO, M. C. ET AL.: 'Stage-specific signaling through TGFbeta family members and WNT regulates patterning and pancreatic specification of human pluripotent stem cells' DEVELOPMENT vol. 138, no. 5, March 2011, pages 861 - 871, XP055063429
- MFOPOU, J. ET AL.: 'Recent advances and prospects in the differentiation of pancreatic cells from human embryonic stem cells' DIABETES vol. 59, no. 9, September 2010, pages 2094 - 2101, XP009138606
- RANJAN, A. ET AL.: 'Endothelial cells in pancreatic islet development and function' ISLETS vol. 1, no. 1, 2009, pages 2 - 9, XP055175526

## Description

### Field

The disclosure relates to methods and compositions for producing NKX6-1 positive pancreatic progenitor cells and functional pancreatic beta cells.

### Background

The ability to generate pancreatic progenitors cells from human pluripotent stem cells (hPSCs; including embryonic stem cells; hESCs and induced pluripotent stem cells; hiPSCs) can provide a source of human endocrine, exocrine and ductal cells for: 1) predictive drug toxicology and drug discovery, 2) transplantation for the treatment of diabetes and 3) modeling normal pancreatic development and diabetes development in vitro. Realizing this requires the ability to reproducibly generate highly enriched populations of pancreatic progenitor cells from different hESC and hiPSC cell lines and to be able to promote their maturation to insulin producing beta cells in vitro and in vivo. Previous publications have reported that it is possible to generate insulin producing cells from hPSCs, however the cells generated were poly-hormonal. Analyses of these insulin producing cells indicates that they do not express NKX6-1, a key transcription factor essential for the development of functional beta cells. One study has demonstrated the generation of NKX6-1+ progenitor cells from hESCs and shown that following transplantation into immunocompromised recipient, they are able to differentiate to functional beta cells **(Kelly et al., 2011).** However, the data in this study is based on two hESC lines that have a propensity to differentiate to the pancreatic lineage. When applied to other lines, the protocol described in the study did not promote the efficient development of NKX6-1+ progenitors **(Kelly et al., 2011).**

### Summary

An aspect of the disclosure describes a method of producing NKX6-1+ pancreatic progenitor cells from an endodermal cell population, the method comprising contacting the endodermal cell population with an EGF component, a Nicotinamide component and/or a Noggin component. An aspect of the method according to the invention provides a method of producing NKX6-1+ pancreatic progenitor cells from an endodermal cell population, the method comprising contacting the endodermal cell population with a combination of 1) at least one EGF component selected from EGF, an active conjugate of EGF and an active fragment of EGF; and 2) nicotinamide or a salt thereof, in an amount sufficient to induce the differentiation of at least a portion of the endodermal cell population into NKX6-1+ pancreatic progenitor cells.

In an embodiment, the EGF component comprises EGF and/or active conjugates and/or fragments thereof; Amphiregulin (AR) and/or active conjugates and/or fragments thereof; Transforming Growth Factor α (TGFα) and/or active conjugate and/or fragments thereof; Betacellulin (BTC) and/or active variants and/or fragments thereof; Epiregulin (EPR) AND/OR active variants and/or fragments thereof; Neuregulins (including for example NRG1, NRG2, 3 NRG4) and/or active variants and/or fragments thereof; and/or combinations of two or more thereof.

In an embodiment, the Nicotinamide component comprises nicotinamide (also known as niacinamide and nicotinic acid amide), salts, solvates and conjugates thereof; Poly(ADP-ribose) synthetase inhibitors such as benzamide and/or 3-aminobenzamide (Otonkoski et al., 1993). Sir-2 inhibitors such as sirtinol, M15, splitomicin (Bitterman et al., 2002); other HDAC inhibitors such as AN-9, CI-994, FK228, LAQ-824, MS275, SAHA, Valproic Acid, TSA, Sodium Butyrate (Bitterman et al., 2002), and/or combinations of two or more thereof.

In an embodiment, the Noggin component comprises one or more of Noggin and/or active conjugates and/or fragments thereof; BMPR1A and/or active conjugates and/or fragments thereof; BMPR1B and/or active conjugates and/or fragments thereof Dorsomorphin and/or salts or solvates, thereof; LDN 193189 also known as "DM-3189" and/or salts and/or solvates thereof; CHORDIN and/or active conjugates and/or fragments thereof; and/or any combination of two or more thereof.

In an embodiment, the combination comprises at least one Noggin component; at least one EGF component and at least one nicotinamide component.

In an embodiment, the combination of at least one Noggin component; at least one EGF component and at least one nicotinamide component comprises Noggin, EGF and nicotinamide (e.g. NENA).

In an embodiment, the method further comprises contacting said cells with an Exendin-4 component.

In an embodiment, the method induces the differentiation of at least 10%, at least 15%, least 20%, at least 25%, at least 30%, at least 40%, at least 45%, at least 50% or at least 55% of the endodermal cell population into a NKX6-1+ pancreatic progenitor cells

In an embodiment, the method first comprises producing an endodermal cell population.

In an embodiment, the method of producing the endodermal cell population comprises one or more of steps (or substeps) a, b and c; and step d, the steps comprising:
a) contacting a pluripotent stem cell population with a combination of:
   I) a nodal agonist, optionally ActA and a wnt signaling agonist, optionally, Wnt3a or CHIR 99021;
   II) a nodal agonist, optionally Act A, a FGF agonist, optionally bFGF and optionally a wnt signaling agonist, optionally Wnt3a CHIR 99021; and
   III) a nodal agonist, optionally ActA and a FGF agonist, optionally bFGF;
   to produce Stage 1 differentiated cells;
b) contacting the Stage I differentiated cells with a FGF agonist, optionally FGF10, and optionally wnt signaling agonist, optionally Wnt3a and/or a noggin component, optionally Dorsomorphin, to produce Stage 2 differentiated cells;
c) contacting the Stage 2 differentiated cells with a Noggin component, optionally Noggin, Retinoic acid (RA) or RA analog, and optionally cyclopamine-KAAD (Cyc), a FGF agonist, optionally FGF10 and/or an Exendin-4 component, optionally Exendin-4 to provide a endodermal cell population; and
d) contacting the endodermal cell population with an EGF component, a Nicotinamide component and/or a Noggin component, optionally a combination comprising at least one EGF component and at least one nicotinamide component in an amount sufficient to induce the differentiation of at least a portion of the endodermal cell population into NKX6-1+ pancreatic progenitor cells.

In an embodiment, the combination to produce the NKX6-1 pancreatic progenitor cells further comprises at least one Noggin component.

Another aspect described herein includes a method for generating NKX6-1+ insulin producing cells comprising:
a) producing a population of cells comprising NKX6-1 positive pancreatic progenitor cells according to a method described herein; and
b) introducing the population of cells, or an NKX6-1 enriched or isolated population into a subject.

A further aspect described herein includes a method for generating insulin producing cells comprising:
a) generating a population of cells comprising NKX6-1 positive pancreatic progenitor cells;
b) co-culturing the NKX6-1 positive pancreatic progenitor cells with CD34+ endothelial cells for a length of time sufficient to obtain insulin producing cells, optionally NKX6-1 positive insulin producing cells.

In an embodiment of the invention, the method further comprises enriching or isolating a NKX6-1 positive population of cells.

In another embodiment of the invention, the enriching or isolating step comprises contacting the population of cells with an HPx epitope detecting antibody and isolating or partially purifying the HPx1 and/or HPx2 bound subpopulation of cells.

Another aspect includes an enriched and/or isolated NKX6-1 positive cell population produced according to the method described herein and a suitable diluent.

In an embodiment, the enriched or isolated NKX6-1 positive cell population comprises at least 10%, at least 15%, at least 20%, at least 25%, at least 30%, at least 35%, at least 40%, at least 50%, at least 60%, at least 70%, at least 80% or up to about 95% NKX6-1 positive cells.

Yet a further aspect described includes a culture medium supplement composition comprising an EGF component, a Nicotinamide component and/or a Noggin component and optionally an Exendin-4 component.

Also provided in another described aspect is a culture medium composition comprising a base medium and a culture medium supplement described herein.

A further described embodiment includes a kit comprising:
a) an EGF component,
b) a Nicotinamide component; and/or.
c) a Noggin component.

Also described are compositions comprising and uses of the population of cells for predictive drug toxicology and drug discovery, transplantation, treating diabetes, tissue engineering and/or modeling disease.

Other features and advantages of the present disclosure will become apparent from the following detailed description. It should be understood, however, that the detailed description and the specific examples while indicating preferred embodiments of the disclosure are given by way of illustration only.

### Brief description of the drawings

An embodiment of the present disclosure will now be described in relation to the drawings in which:
**Figure 1** is a schematic of an example of a pancreatic differentiation protocol;
**Figure 2** is a graph that demonstrates the percentage of NKX6-1:GFP+ cells induced from hESC derived cells after treatment with Noggin, EGF, Nicotinamide (NENA) an Exendin-4;
**Figure** 3 is a series of graphs that demonstrates mRNA expression levels of NKX6-1, PDX1, PTF1A and SOX9 in a sample study;
**Figure 4A** is a graph that demonstrates that using the NKX6-1^{*GFP*/*w*} HESC line, the percentage of NKX6-1:GFP+ cells is highest when endodermal cells are cultured in the presence of Noggin, EGF, Nicotinamide (with or without Exendin-4), compared to no treatment or various combination of the above compounds.
**Figure 4B** is a graph that demonstrates that using the H1 (WA01) HESC line, the percentage of NKX6-1+ cells is highest when endodermal cells are cultured in the presence of Noggin, EGF, Nicotinamide (with or without Exendin-4), compared to no treatment or various combination of the above compounds.
**Figure** 5 is a series of images showing that NKX6-1 cells generated using the "NENA treatment" have the potential to generate insulin-producing cells. Immunohistochemical analysis of NKX6-1 and INSULIN in the mammary fat pad harvested 6 weeks post transplantation of H1 (WA01) d14 differentiated cells (A). Immunohistochemical analysis of C-peptide and GLUCAGON (GCG) in the mammary fat pad harvested 6 weeks post transplantation of H1 (WA01) d14 differentiated cells (B).
**Figure 6** is a series of flow cytometric profiles showing that HPx can be used to enrich for NKX6-1 expressing cells. Flow cytometric analysis for NKX6-1:GFP alone (A), versus hPx (B) and versus CD142 (C) at day 13 of differentiation of NKX6-1^{*GFP*/*w*} hESC line cultured in the presence or absence of the triple treatment (Noggin, EGF and Nicotinamide).
**Figure 7A** is a graph that demonstrates that isolating HPx2+ cells using fluorescence activated cell sorting (FACS) enriches for a population that expresses higher levels of NKX6-1 mRNA compared to unsorted cells (PS=Presort), similarly to the levels found in the CD142-enriched (CD142+) population.
**Figure 7B** is a graph that demonstrates that isolating HPx2+CD142+ double positive cells using fluorescence activated cell sorting (FACS) enriches for a population that expresses higher levels of NKX6-1 mRNA compared to unsorted cells (PS=Presort),
**Figure 8A** is a series of flow cytometric profiles of day 6 mesoderm;
**Figure 8B** is a phase picture of CD34+ cells.
**Figures 8C-D** are images of cells showing Acetylated LDL uptake;
**Figure 8E** is an image showing immunohistochemistry for vWF.
**Figure 9A** is a series of plots top panel: FACS analysis for GFP in live cells from day 14 cultures as control monolayer (Ctrl), aggregates on collagen (Col) and co-culture with ES-derived endothelial cells (ES-E). Bottom panel: Intra-cellular FACS for c-peptide within the GFP+ population from each culture condition.
**Figure 9B** is a graph showing real time QPCR analysis for Insulin (INS) and Glucagon (GCG) under different culture condition. Numbers are relative to FOXA2.
**Figure 10** is a graph that demonstrates that isolating HPx1+ cells using fluorescence activated cell sorting (FACS) enriches for a population that expresses higher levels of NKX6-1, SOX9, and PTF1a mRNA compared to HPx1- cells.
**Figure 11** is a graph that demonstrates that NKX6-1 cells generated using the "NENA" treatment have the potential to generate functional insulin producing cells. Human C-peptide secretion in response to glucose challenge was measured by ELISA at 6, 12 and 18 weeks post-transplantation of H1 (WA01) d13 differentiated cells into immunocompromised mice.

### Detailed description of the Disclosure

Described herein is a robust and reliable platform for the efficient generation of NKX6-1+ pancreatic progenitor cells from human pluripotent stem cells (hPSC), using a defined combination of factors including BMP inhibitors (e.g such as Noggin), nicotinamide and agonists of the EGF signaling pathway. Monohormonal insulin producing beta cells are NKX6-1 positive, similar to the cells produced herein. It is demonstrated herein using mouse transplantation experiments that the cells produced according to the methods described are monohormonal and functional

It is also demonstrated herein that NKX6-1+ pancreatic endocrine progenitor cells can be differentiated from different populations of human pluripotent stem cells (hPSCs).

An aspect of the present disclosure includes a method of producing NKX6-1+ pancreatic progenitor cells from an endodermal cell population the method comprising contacting the endodermal cell population with an EGF component, a Nicotinamide component and/or a Noggin component, optionally a combination of at least one EGF component and at least one nicotinamide component, to induce the differentiation of at least a portion of the endodermal cell population into NKX6-1+ pancreatic progenitor cells. An aspect of the present invention provides a method of producing NKX6-1+ pancreatic progenitor cells from an endodermal cell population, the method comprising contacting the endodermal cell population with a combination of 1) at least one EGF component selected from EGF, an active conjugate of EGF and an active fragment of EGF; and 2) nicotinamide or a salt thereof to induce the differentiation of at least a portion of the endodermal cell population into NKX6-1+ pancreatic progenitor cells.

In an embodiment, the combination further comprises at least one Noggin component.

The term "contacting" (e.g. contacting an endodermal cell population with a component or components) is intended to include incubating the component(s) and the cell together in vitro (e.g., adding the compound to cells in culture) and the step of contacting can be conducted in any suitable manner. For example the cells may be treated in adherent culture, or in suspension culture, the components can be added temporally substantially simultaneously (e.g. together in a cocktail) or sequentially (e.g. within 1 hour from an addition of a first component). The cells can also be contacted with another agent such as a growth factor or other differentiation agent or environments to stabilize the cells, or to differentiate the cells further and include culturing the cells under conditions known in the art for example for culturing the pluripotent (and/or differentiated) population for example as further described in the Examples.

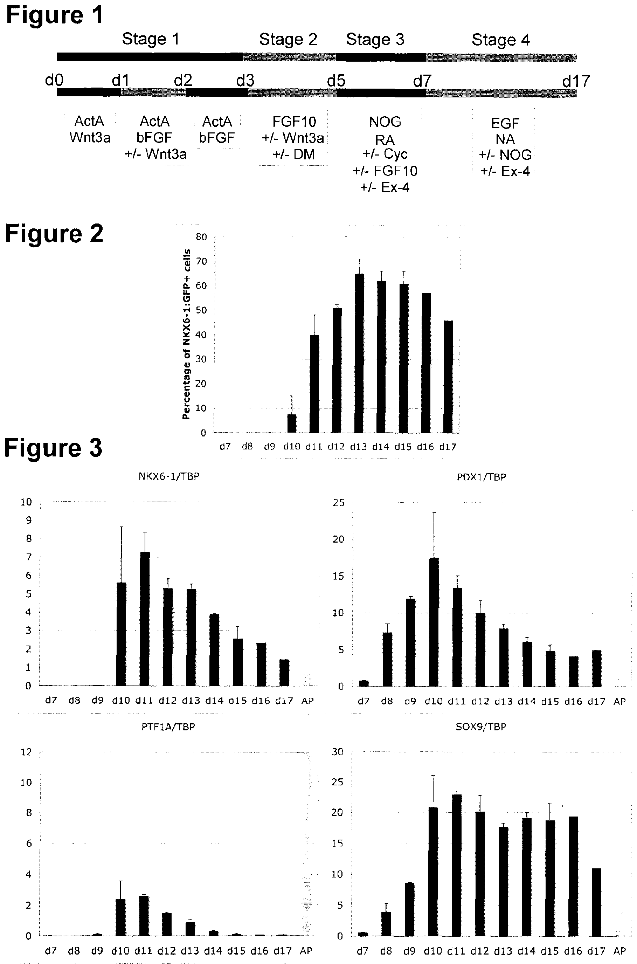

The "endodermal cell population" as used herein refers to a population of cells comprising at least 20%, at least 30%, at least 40%, at least 50%, at least 60%, at least 70%, at least 80%, at least 90% endoderm cells corresponding to Stage 3 of Figure 1. Endoderm cells correspond for example to an embryonic posterior foregut cell population which express at least FOXA2, and can be positive or negative for PDX1. Other factors can also be expressed. The population can be NKX6-1 negative (NKX6-1-) or express low levels of NKX6-1. The endodermal cell population can for example be identified by flow cytometric and molecular analysis for one or more markers such as FOXA2. The endodermal cell population can for example be in a 2D (monolayer) or 3D (Embryoid Body or other form of aggregates) formats.

The term "endoderm" as used herein refers to one of the three primary germ cell layers in the very early embryo (the other two germ cell layers are the mesoderm and ectoderm). The endoderm is the innermost of the three layers. An endoderm cell differentiates to give rise first to the embryonic gut and then to the lining of the gut (esophagus, stomach, intestine, rectum, colon), pharyngeal pouch derivatives (tonsils, thyroid, thymus, parathyroid glands), lung, liver, gall bladder and pancreas.

The term "pluripotent stem cell" as used herein refers to a cell with the capacity, under different conditions, to differentiate to more than one differentiated cell type, and for example the capacity to differentiate to cell types characteristic of the three germ cell layers, and includes embryonic stem cells and induced pluripotent stem cells. Pluripotent cells are characterized by their ability to differentiate to more than one cell type using, for example, a nude mouse teratoma formation assay. Pluripotency is also evidenced by the expression of embryonic stem (ES) cell marker.

The term "progenitor cell" refers to cells that have a cellular phenotype that is at an earlier step along a developmental pathway or progression than is a fully differentiated cell relative to a cell which it can give rise to by differentiation. Progenitor cells can give rise to multiple distinct differentiated cell types or to a single differentiated cell type, depending on the developmental pathway and on the environment in which the cells develop and differentiate.

The term "pancreatic progenitor cell" refers to a cell which is capable of forming any of: pancreatic endocrine cells (such as glucagon producing alpha cells or insulin producing beta cells), or pancreatic exocrine cells (e. g. amylase + and/or trypsin + pancreatic cells) or pancreatic duct cells. Formation or development of one more of pancreatic endocrine cells, pancreatic exocrine cells or pancreatic duct cells may be induced by addition of components such as a combination of an EGF component and a nicotinamide component and/or resulting from the environment in which the progenitor cell develops. For example a "NKX6-1 positive pancreatic pluripotent progenitor cell" under conditions including endothelial contact can give rise to functional beta cells. Similarly injection of NKX6-1 pancreatic progenitor cells in vivo can result in development of such cells. NKX6-1 pancreatic progenitor cells have also been demonstrated to give rise to insulin producing NKX6-1 positive beta cells and CK-19 positive ductal cells as described in the Examples.

The term "functional beta cell" as used herein means a type of pancreatic cell, which in the pancreas is located in areas called the islets of Langerhans, that is NKX6-1 positive and/or PDX1 positive and makes and releases insulin.

The term "stem cell" as used herein, refers to an undifferentiated cell which is capable of proliferation, self-renewal and giving rise to more progenitor cells having the ability to generate a large number of mother cells that can in turn give rise to differentiated or differentiable daughter cells. The daughter cells can for example be induced to proliferate and produce progeny that subsequently differentiate into one or more mature cell types, while also retaining one or more cells with parental developmental potential.

In the context of a cell, the term "differentiated", or "differentiating" is a relative term and a "differentiated cell" is a cell that has progressed further down the developmental pathway than the cell it is being compared with. Thus, stem cells can differentiate to lineage-restricted precursor cells (such as a endodermal progenitor cell), which in turn can differentiate into other types of precursor cells further down the pathway and then to an end-stage differentiated cell, which plays a characteristic role in a certain tissue type, and may or may not retain the capacity to proliferate further.

The term "embryonic stem cell" is used to refer to the pluripotent stem cells which can be obtained from the inner cell mass of the embryonic blastocyst (see, for example, U.S. Pat. Nos. 5,843,780, 6,200,806). Such cells can also be obtained from the inner cell mass of blastocysts (see, for example, U.S. Pat. Nos. 5,945,577, 5,994,619, 6,235,970). The distinguishing characteristics of an embryonic stem cell define an embryonic stem cell phenotype. Accordingly, a cell has the phenotype of an embryonic stem cell if it possesses one or more of the unique characteristics of an embryonic stem cell such that that cell can be distinguished from other cells. Exemplary distinguishing embryonic stem cell characteristics include, without limitation, gene expression profile, proliferative capacity, differentiation capacity, karyotype, responsiveness to particular culture conditions, and the like.

The term "expression" refers to the cellular processes involved in producing RNA and proteins and as appropriate, secreting proteins, including where applicable, but not limited to, for example, transcription, translation, folding, modification and processing. "Expression products" include RNA transcribed from a gene and polypeptides obtained by translation of mRNA transcribed from a gene.

The term "NKX6-1 positive pancreatic progenitor cell" as used herein refers to a cell which is derived for example from a pancreatic endoderm cell and which has the capacity to differentiate at least into insulin producing cells, such as pancreatic beta-cells. A NKX6-1 positive pancreatic progenitor expresses the marker NKX6-1 and can express for example increased levels of PDX1, PTF1A and SOX9 compared to a pluripotent stem cell (PSC).

The term "NKX6-1" as used herein refers to the mRNA (and/or optionally cDNA) or protein product of the "NK6 homeobox 1" gene (Gene ID: 4825), the sequences, including protein and mRNA sequences (and/or optionally cDNA).

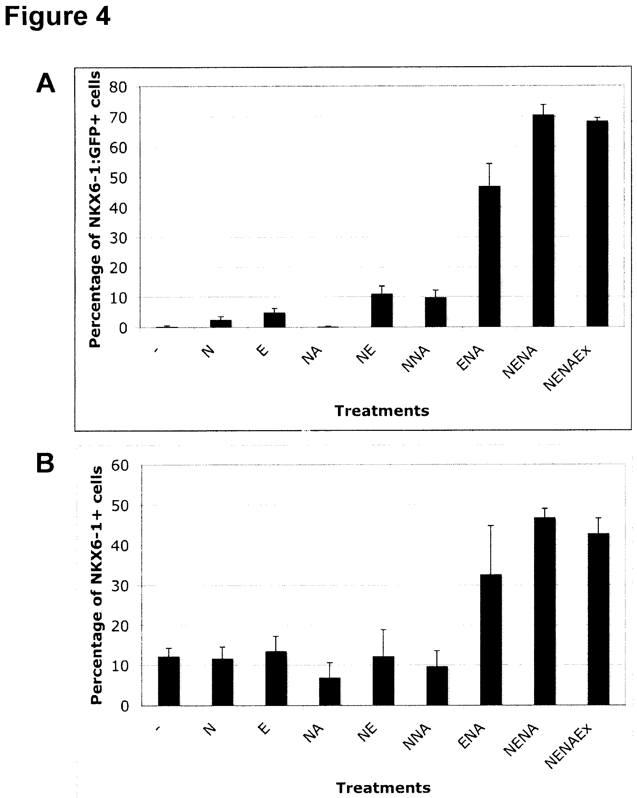

As demonstrated herein, treatment with EGF and Nicotinamine gives rise to NKX6-1+ cells which are increased by the addition of Noggin (Figure 4).

Accordingly in a further embodiment, the combination further comprises a Noggin component.

The term "Noggin component" as used herein means any polypeptide or compound BMP antagonist that inhibits TGF-beta/BMP family signal transduction for example by binding a TGF beta family ligand, including but not limited to agents provided in Example 10, including for example Noggin (NOG) such as human Noggin having Gene Identification number **(Gene ID: 9241)** as well as active conjugates and fragments thereof, including naturally occurring active conjugates and fragments; bone morphogenetic protein receptor, type IA (BMPR1A), for example human BMPR1A having for example Gene Identification number **(Gene ID: 657)** as well as active conjugates and fragments thereof, including naturally occurring active conjugates and fragments; bone morphogenetic protein receptor, type IB (BMPR1B), for example human BMPR1B having for example Gene Identification number **(Gene ID: 658)** as well as active conjugates and fragments thereof, including naturally occurring active conjugates and fragments; small molecule chemical inhibitor Dorsomorphin and salts, solvates and combinations thereof; small molecule chemical inhibitor LDN 193189 also known as "DM-3189" and/or salts and/or solvates thereof; and CHORDIN, for example human CHORDIN (CHRD) having for example Gene Identification number **(Gene ID: 8646)** as well as active conjugates and fragments thereof, including naturally occurring active conjugates and fragments; and/or combinations of two or more of the above, including for example combinations comprising Noggin. The sequences, including protein and mRNA sequences, of each (e.g. each component identified by Gene ID) are encompassed by the term.

The term "Noggin" as used herein refers to a bone morphogenetic protein antagonist having Gene Identification number **(Gene ID: 9241),** the sequences, including protein and mRNA sequences are encompassed by the term.

The compound "Dorsomorphin" includes compounds of the formula: 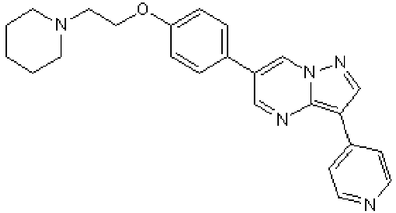 salts, solvates and/or combinations thereof.

The compound "LDN" means compounds having the formula: 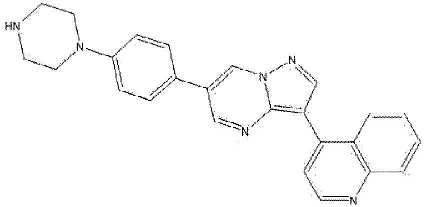 salts, solvates and/or combinations thereof.

In an embodiment, the Noggin component is Noggin. The concentration of Noggin can for example range from about 1 ng to about 500 ng/ml for example from about 1 ng to about 250 ng/ml, from about 10 ng to about 250 ng/ml from about 10 ng to about 100 ng/ml. In another embodiment, the Noggin concentration is about 10 ng/ml, about 20 ng/ml, about 30 ng/ml, about 40 ng/ml, about 50 ng/ml, about 60 ng/ml, about 70 ng/ml, about 80 ng/ml, about 90 ng/ml, about 100 ng/ml, about 150 ng/ml, about 200 ng/ml, about 300 ng/ml, about 400 ng/ml, or about 500 ng/ml.

The term "EGF component" as used herein means any polypeptide or small molecule that activates any of the EGF receptor family members (ErbB1/HER-1/EGFR, gene ID:1956, ErbB2/HER-2/neu, gene ID:2064 ErbB3/HER-3, gene ID: 2065, and/or ErbB4/HER-4, gene ID:2066) including for example but not limited to agents provided in Example 11, epidermal growth factor (EGF), for example human EGF having for example Gene Identification number **(Gene ID: 1950)** as well as active conjugates and fragments thereof, including naturally occurring active conjugates and fragments; Amphiregulin (AR) for example human AR/AREG having for example Gene Identification number **(Gene ID: 374)** as well as active conjugates and fragments thereof, including naturally occurring active conjugates and fragments; Transforming Growth Factor α (TGFα) for example human TGFα having for example Gene Identification number **(Gene ID: 7039),** as well as active conjugate and fragments thereof, including naturally occurring active conjugates and fragments; Betacellulin (BTC) for example human BTC having for example Gene Identification number **(Gene ID: 685)** as well as active variants and fragments thereof, including naturally occurring active conjugates and fragments; Heparin-binding EGF-like growth factor (HB-EGF) having for example Gene Identification number **(Gene ID: 1839),** as well as active conjugate and fragments thereof, including naturally occurring active conjugates and fragments; Epiregulin (EREG/ER) for example human EREG having for example Gene Identification number **(Gene ID: 2069),** as well as active variants and fragments thereof, including naturally occurring active variants and fragments; Neuregulins (including for example NRG1, NRG2, NRG3 NRG4) for example human NRGs having for example Gene Identification number **(Gene ID: 3084, Gene ID: 9542, Gene ID: 10718, Gene ID: 145957,** respectively), as well as active variants and fragments thereof, including naturally occurring active variants and fragments and/or combinations of two or more of the above, for example a combination comprising human EGF.

In an embodiment, the term "EGF" as used herein refers to Epidermal growth (EGF), for example human EGF having for example Gene Identification number **(Gene ID: 1950)** as well as active conjugates and fragments thereof, including naturally occurring active conjugates and fragments.

In an embodiment, the EGF component is EGF. The concentration of EGF can for example range from about 1 ng to about 500 ng/ml for example from about 1 ng to about 250 ng/ml, from about 10 ng to about 250 ng/ml from about 10 ng to about 100 ng/ml. In another embodiment, the EGF concentration is about 10 ng/ml, about 20 ng/ml, about 30 ng/ml, about 40 ng/ml, about 50 ng/ml, about 60 ng/ml, about 70 ng/ml, about 80 ng/ml, about 90 ng/ml, about 100 ng/ml, about 150 ng/ml, about 200 ng/ml, about 300 ng/ml, about 400 ng/ml, or about 500 ng/ml.

The term "nicotinamide component" as used herein means any inhibitor of sirtuin histone deacetylases (HDACs) including but not limited to nicotinamide (also known as niacinamide and nicotinic acid amide), salts, solvates and conjugates thereof; Poly(ADP-ribose) synthetase inhibitors such as benzamide and/or 3-aminobenzamide (Otonkoski et al., 1993), Sir-2 inhibitors such as sirtinol, M15, splitomicin (Bitterman et al., 2002); other HDAC inhibitors such as AN-9, CI-994, FK228, LAQ-824, MS275, SAHA, Valproic Acid, TSA, Sodium Butyrate (Bitterman et al., 2002), and/or combinations of two or more thereof, for example combinations including nicotinamide. For example, Bitterman et al, 2002 and Otonkoski et al 1993 demonstrate that sirtinol, M15, splitomicin benzamide and/or 3-aminobenzamide have a similar or better effect than nicotinamide.

The term "Nicotinamide" as used herein refers to a water-soluble vitamin also known as niacinamide and nicotinic acid amide as well as salts, solvates and conjugates thereof, and is the amide of nicotinic acid (vitamin B3/niacin).

In an embodiment, the Nicotinamide component is Nicotinamide and/or a salt, solvate and/or conjugate thereof. The concentration of Nicotinamide can for example range from about 10 µM to about 100 mM for example from about 10 µM to about 50 mM, from about 1 mM to about 100 mM, or from about 1 mM to about 50 mM. In another embodiment, the Nicotinamide concentration is about 1 mM, about 2 mM, about 5 mM, about 10 mM, about 15 mM, about 20 mM, about 30 mM, about 40 mM, about 50 mM, about 60 mM, about 70 mM, about 80 mM, about 90 mM, or about 100 mM.

It is demonstrated herein in Example 13 for example, that treating cells with a combination of Noggin, EGF and Nicotinamide can induce greater than 10% of treated cells to express NKX6-1.

Accordingly in an embodiment, the combination of at least one Noggin component; at least one EGF component and at least one nicotinamide component comprises Noggin, EGF and nicotinamide (e.g. NENA).

Nicotinamide has been shown to be an potent inducer of endocrine differentiation in human fetal pancreatic cells (Otonkoski et al.,1993). However, prior to this disclosure it has not been used in combination with BMP inhibitors and/or agonists of the EGF pathway (EGF or other EGF-related growth factors) to specifically promote the development of NKX6-1⁺ progenitor cells from definitive endoderm. Inclusion of nicotinamide for example improves the reproducible and efficient production of NKX6-1⁺ pancreatic progenitors from different hPSC lines.

Exendin-4 and active conjugates and fragments thereof can also be used to induce pancreatic progenitor differentiation. Example 13 also shows that a combination comprising Exendin-4 induces differentiation in at least 30% of the endodermal cell population.

Accordingly, in an embodiment, the combination of at least one Noggin component; at least one EGF component and at least one nicotinamide component further comprises an Exendin-4 component.

The term "Exendin-4 component" as used herein refers to polypeptides and compounds that activate GLP-1 (Glucagon-like peptide-1) receptors to increase intracellular cAMP, including for example but not limited to Exendin-4 (SIGMA 7144), and active conjugates and fragments thereof, GLP-1 and active conjugates and fragments thereof such as human Glucagon like peptide-1 (SIGMA G3265) and Glucagon-like peptide Amide Fragment 7-36 human (SIGMA G8147), as well as active conjugates and fragments thereof, including naturally occurring active conjugates and fragments.

Variants such as conservative mutant variants and activating mutant variants for each of the polypeptides can also be used. For example, activating mutants of GLP-1 such as GLP-1 (7-37) A8G, can also be included.

The term "Exendin-4" as used herein refers to a 39 amino acid peptide and active conjugates and fragments thereof that activate GLP-1 (Glucagon-like peptide-1) receptors to increase intracellular cAMP (SIGMA 7144).

The term "active fragments" as used herein is a polypeptide having amino acid sequence which is smaller in size than, but substantially homologous to the polypeptide it is a fragment of, and where the active fragment polypeptide is about at least 50%, or 60% or 70% or at 80% or 90% or 100% or greater than 100%, for example 1.5-fold, 2-fold, 3-fold, 4-fold or greater than 4-fold as effective in terms of biological action as the polypeptide from which it is a fragment of. Examples include fragments of EGF which bind and activate EGF receptor, and human Glucagon-like peptide Amide Fragment 7-36 (GLP-1 7-36 amide) (SIGMA G8147) which is like GLP-1 7-37 also effective for stimulating insulin production but not inactive fragments such as GLP-1 9-37.

In an embodiment, the combination of at least one Noggin component; at least one EGF component and at least one nicotinamide component further comprises at least one Exendin-4 component comprises Noggin, EGF, Nicotinamide and Exendin-4 (e.g. NENAEx).

In an embodiment, the endodermal cell population is contacted with the combination of at least one Noggin component, at least one EGF component, at least one Nicotinamide component and optionally at least one Exendin-4 component for at least or about 3 days, at least about 4 day, at least about 5 day, at least about 6 day, at least about 7 day, at least about 8 day, at least about 9 day, or at least about 10 days. In another embodiment, the endodermal cell population is contacted with the combination for at least about 11 days, at least about 12 days, at least about 13 days, at least about 14 days, or at least about 15 days. It is found that NKX6-1 positivity and/or capacity to generate insulin producing cells can persist for example at least 30 days or more in cultured cells.

The differentiation of at least 10%, at least 15%, at least 20%, at least 25%, at least 30%, at least 35%, at least 40%, at least 45% or at least 50% or at least 60% or at least 70% or at least 80%, at least 90%, of the endodermal cell population is induced to differentiate into a NKX6-1+ pancreatic progenitor cells.

Differentiation can be detected by determining the level of pancreatic progenitor markers. For example, NKX6-1, PDX1, PTF1A, and SOX9 are pancreatic progenitor markers whose mRNA expression can be detected for example by RT-PCR. Differentiation can also be detected using antibodies that recognize pancreatic progenitor cells. As described herein, anti-HPx1 and anti-HPx2 antibodies which had been shown to react with cell surface markers of adult human pancreatic exocrine cells, can also be used to monitor the development of NKX6-1+ pancreatic progenitor cells, similarly for example to anti CD142 antibody (Kelly et al., 2011).

In an embodiment, the endodermal cell population is differentiated from pluripotent stem cells (PSCs) such as an embryonic stem cell (ESC) or an induced pluripotent stem cells (iPSCs).

In an embodiment, the pluripotent stem cell is from a mammal, such as a human. In an embodiment, the pluripotent stem cell is a human ESC (hESC) or a human iPSC (hiPSC).

As used herein, the terms "iPSC" and "induced pluripotent stem cell" are used interchangeably and refers to a pluripotent stem cell artificially derived (e.g., induced or by complete reversal) from a non-pluripotent cell, typically an adult somatic cell, for example, by inducing expression of one or more genes (including POU4F1/OCT4 (Gene ID; 5460) in combination with, but not restricted to, SOX2 (Gene ID; 6657), KLF4 (Gene ID; 9314), cMYC (Gene ID; 4609), NANOG (Gene ID; 79923), LIN28/ LIN28A (Gene ID; 79727)).

In an embodiment, the method comprises steps for obtaining the endodermal cell population. For example, as described herein differentiation from a pluripotent stem cell such as an ESC or an iPSC to an endodermal cell involves a series of steps that is characterized in Figure 1 as comprising Stages 1 to 3. Stage 1 can further be divided into substages for example three substages spanning for example days 0 to 5. Stage 2 can span for example 2 to 3 days and stage 3 can span for example 1 to 4 days. As described in Nostro et al 2011 and herein, Stage 1 can comprise contacting a population of pluripotent stems cells with ActA (or other Nodal agonist) and Wnt3a at day 0, contacting the population with Act A, bFGF and optionally Wnt3a at day 1 and contacting the population with ActA and bFGF at day 2 to produce Stage 1 differentiated cells. Stage 2 for example can include contacting the population to be differentiated (e.g. the Stage 1 differentiated cells) with Fibroblast Growth Factor 10 (FGF10), optionally wingless-type MMTV integration site family member 3A (Wnt3a) and optionally Dorsomorphin to produce Stage 2 differentiated cells. Stage 3 for example can include contacting the population to be differentiated (e.g. the Stage 2 differentiated cells) with Noggin, optionally cyclopamine-KAAD (Cyc) (or any other hedgehog (HH) signaling inhibitors), Retinoic acid (RA), optionally FGF10 and/or optionally Exendin-4 to provide an endodermal cell population. Dorsomorphin can be substituted for example with other noggin components such as chordin LDN 193189 and BMPRs; ActA can be substituted with other Nodal agonists and Wnt 3a can be substituted with other Wnt signaling agonists such as or other wnt/beta catenin agonist such as CHIR99021. Similarly, bFGF and FGF10 can be substituted with other FGFs or compounds that activate the same receptor as bFGF or FGF10 respectively. Retinoic acid can be substituted for example by a retinoic acid analog. The protocol can for example be the protocol previously described (Nostro et al., 2011), and/or with modifications (e.g. addition of Wnt3a at day 1 and Exendin-4, Ex-4 from day 5 to day 7). Variations include for example, omitting FGF10 and/or Exendin-4 from the protocol and using for example CHIR99021 (Stemgent 04-0004) as replacement for Wnt3a. Protocols for generating Stage 3 cells are described for example in US Patents 7,989,204, 7,993,916, 8,129,182 and 8,187,878. Methods described therein can be used to obtain Stage 3 cells which are further differentiated using any of the Stage 4 methods described herein (e.g. ENA, NENA, NExENA etc).

ActA is a nodal agonist. The term "nodal agonist" as used herein means any molecule that activates nodal signal transduction such as "nodal" (for example human nodal such as Gene ID: 4338) or "activin". The term "activin" or "ActA" as used herein refers to "Activin A" (for example Gene ID: 3624), for example human activin, as well as active conjugates and fragments thereof, optionally including naturally occuring active conjugates and fragments, that can for example activate nodal signal transduction as well as active conjugates and fragments thereof, including naturally occuring active conjugates and fragments.

Wnt3A is a wnt signaling agonist. The term "a wnt signaling agonist" as used herein means any molecule that activates wnt/beta-catenin receptor signaling in a hepatocyte and incldues for example Wnt3a and as well as GSK3 selective inhibitors such as CHIR99021 (Stemolecule™ CHIR99021 Stemgent), 6-Bromolndirubin-3'-Oxime (BIO) (Cayman Chemical (cat:13123)), or Stemolecule™ BIO from Stemgent (cat:04003). CHIR99021 is a selective inhibitor of GSK3. The GSK3 selective inhibitors contemplated are for example selective inhibitors for GSK-3α/β in the Wnt signaling pathway.

FGF10 and bFGF are FGF members that activate FGF receptor signaling.

The term "FGF agonist" as used herein means a molecule such as a cytokine, including for example FGF, or a small molecule, that activates a FGF signalling pathway, e.g binds and activates a FGF receptor. The term "FGF" as used herein refers to any fibroblast growth factor, for example human FGF1 (Gene ID: 2246), FGF2 (also known as bFGF; Gene ID: 2247), FGF3 (Gene ID: 2248), FGF4 (Gene ID: 2249), FGF5 (Gene ID: 2250), FGF6 (Gene ID: 2251), FGF7 (Gene ID: 2252), FGF8 (Gene ID: 2253), FGF9 (Gene ID: 2254) and FGF10 (Gene ID: 2255) optionally including active conjugates and fragments thereof, including naturally occuring active conjugates and fragments. In certain embodiments, FGF is bFGF, FGF10, FGF4 and/or FGF2.

Exemplary ranges for added components for differentiating pluripotent stem cells up to stage 3 (e.g. d7) are provided as previously described (Nostro et al., 2011), and/or cytokines and small molecules can be used at the following concentrations: Activin A (Act1: 10-1000 ng/ml), Wnt3a (Stage 1: 25 ng/ml), CHIR99021 (Stage 1: 0.1-3 µM), bFGF (0.1-50 ng/ml), FGF10 (5-500 ng/ml), Wnt3a (Stage 2: 3 ng/ml), Dorsomorphin (DM: 0.25-0.75 µM), Noggin (NOG: 1-500 ng/ml), Cyclopamine-KAAD (Cyc: 0.5-2.5 µM), Retinoic Acid (RA: 0.02-2 µM) Exendin-4 (Ex-4: 5-500 ng/ml) EGF (1-500 ng/ml), Nicotinamide (NA: 1-100 mM).

Other methods of differentiating cells to obtain an endodermal cell population can also be used.

Accordingly, a further aspect described herein includes a method of producing NKX6-1+ pancreatic progenitor cells from a pluripotent stem cell population the method comprising one or more of steps (or substeps) a, b and c and step d, the steps comprising:
a) contacting a pluripotent stem cell population with a combination of:
   I) a nodal agonist, optionally ActA and a wnt signaling agonist, optionally, Wnt3a or CIHR 99021,
   II) a nodal agonist, optionally Act A, a FGF agonist, optionally bFGF and optionally a wnt signaling agonist, optionally Wnt3a CIHR 99021; and
   III) a nodal agonist, optionally ActA and a FGF agonist, optionally bFGF;
   to produce Stage 1 differentiated cells;
b) contacting the Stage I differentiated cells with a FGF agonist, optionally FGF10, and optionally wnt signaling agonist, optionally Wnt3a and a noggin component, optionally Dorsomorphin, to produce Stage 2 differentiated cells;
c) contacting the Stage 2 differentiated cells with a Noggin , Retinoic acid (RA) or RA analog, and optionally cyclopamine-KAAD (Cyc), a FGF agonist, optionally FGF10 and/or an Exendin-4 component, optionally Exendin-4 to provide a endodermal cell population; and
d) contacting the endodermal cell population with an EGF component, a Nicotinamide component and/or a Noggin component, optionally a combination comprising at least one EGF component and at least one nicotinamide component in an amount sufficient to induce the differentiation of at least a portion of the endodermal cell population into NKX6-1+ pancreatic progenitor cells.

In an embodiment, Stage 2 (optionally instead of Dorsomorphin) and/or further comprises contacting the population with at least one Noggin component.

In an embodiment, the steps comprise two or more of steps of steps a, b and c. For example, a person skilled in the art would recognize that the method to be applied to a Stage 2 cell or equivalent thereof would include steps c) and d). Similarly, one or more substeps of a) can be performed and/or excluded depending on the substage of the starting cell population.

A "Stage 1 cell" as used herein means an endoderm cell characterized at least by the expression of SRY-box containing gene 17 (SOX17) (Gene ID:64321) and chemokine (C-X-C motif) receptor 4 (CXCR4) (Gene ID:7852) generated following in vitro differentiation of human pluripotent stem cells (hESCs and hiPSCs) in 2D or 3D formats, as described for example in Nostro et al., 2011.

A "Stage 2 cell" as used herein means an endoderm cell characterized at least by the expression of forkhead box A2 (FOXA2) (Gene ID:3170) and by the expression of HNF1 homeobox B (HNF1B) (Gene ID:6928) generated following in vitro differentiation of human pluripotent stem cells (hESCs and hiPSCs) in 2D or 3D formats, for example as described in Nostro et al., 2011.

A "Stage 3 cell" as used herein means an endoderm cell characterized at least by the expression of forkhead box A2 (FOXA2) (Gene ID:3170) generated following in vitro differentiation of human pluripotent stem cells (hESCs and hiPSCs) in 2D or 3D formats, for example as described in Nostro et al., 2011.

A "Stage 4 cell" as used herein means an endoderm cell characterized at least by the expression of NK6 homeobox 1 (NKX6-1) (Gene ID:4825) and by the expression of pancreatic and duodenal homeobox 1 (PDX1) (Gene ID:3651) generated following in vitro differentiation of human pluripotent stem cells (hESCs and hiPSCs) as described for example in Example 1.

The NKX6-1 positive progenitor cells can for example be used to generate insulin producing cells in vivo and/or in vitro.

For example, transplanted NKX6-1 positive progenitor cells produced according to a method described herein differentiate to insulin producing cells as demonstrated herein in Example 3.

In an embodiment, cells, for example stage 3 cells, are cultured with a PKC activator, such as indolactam V and/or PDBu. Other compounds can include ITS (i.e. insulin selenium transferrin which is similar to using B27 supplement); CYP26A inhibitor, optionally N-{4-[2-Ethyl-I-(IH-1,2,4-triazol-I-yl)butyl]phenyl}-I,3-benzothiazol-2-amine.

Also provided in another aspect is an in vitro method to produce insulin-producing cells from the Stage 3 endoderm cells and/or from the Stage 4 pancreatic precursor cells, the method comprising co-culturing Stage 3, and/or 4 cells and/or with endothelial cells. In an embodiment, the method comprises: Culturing endodermal cells in presence of CD34+ endothelial cells.

The co-culturing is for example performed in NENA and/or NENaEx media supplemented with bFGF (e.g. 50 ng/ml) and VEGF (100 ng/ml), but can also be performed in any media that is permissive for both pancreatic and endothelial cell survival such as EndoGRO (Millipore), MV2 medium (Promocell), BD endothelial cell culture medium. The medium optionally comprises one or more of a Noggin component, an EGF component, a Nicontinamide component and an Exendin-4 component as well as one or more of bFGF (or other FGF) and VEGF (or other compound that activate signaling through VEGF receptor KDR). In an embodiment, the medium comprises a Noggin component, an EGF component, a Nicontinamide component, bFGF and VEGF. In an embodiment, pancreatic progenitor cells are aggregated. For aggregation of pancreatic progenitors, monolayer cultures can be broken apart mechanically by pipetting, or can be dissociated enzymatically and aggregated with an incubation in low-attachment plates or by shaking the population of cells.

In an embodiment, NKX6-1 positive pancreatic progenitor cells are aggregated and the aggregates are cocultured with CD34+ endothelial cells. The aggregates can be seeded directly on the CD34+ endothelial cells or separated by a biomaterial that allows for diffusion, such as a membrane or other device. The cultures can be maintained for example for an additional 7, 8, 9, 10 or more days optionally in NENAEx-containing media, optionally with 5ng/ml bFGF and 100ng/ml VEGF.

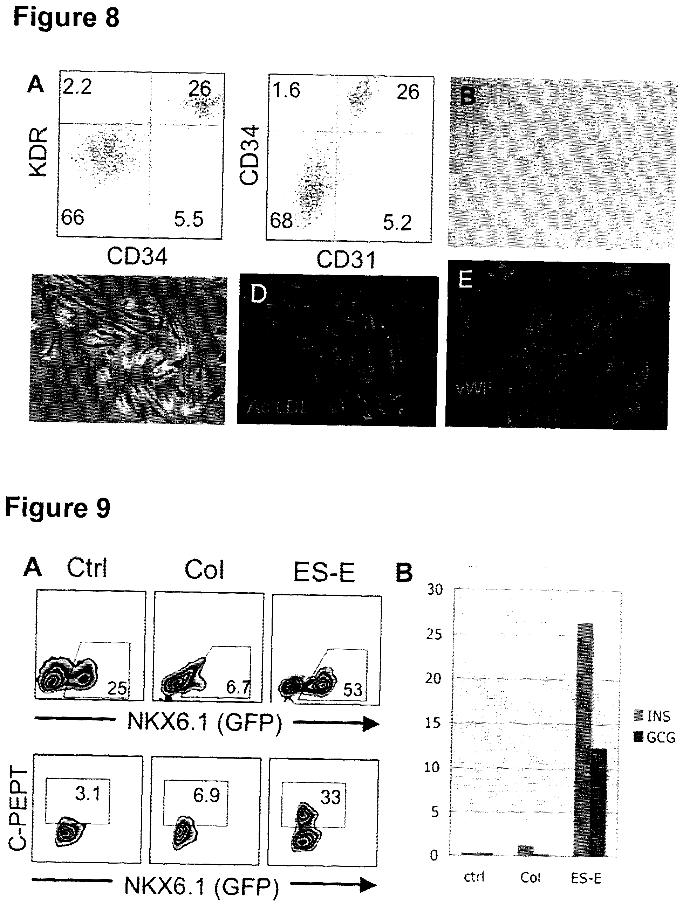

The endothelial cells can for example be hESC derived. For example, hESCs in culture can be induced with a combination of one or more of BMP4, bFGF and VEGF. After a sufficient time, for example 6 days, CD34+ cells can be sorted and cultured in a suitable medium such as EGM-2 medium (Lonza) supplemented for example with bFGF and VEGF. A sufficient time for example, comprises an induction time sufficiently long to induce one or more of KDR, CD31, VE-Cadherin, VEGF receptors, and vWF and/or comprise the capacity to take up acetylated LDL as shown for example in Figure 8.

In an embodiment, the endothelial cells are derived from H1 hESC line. The protocol can be extended to any hESC line and hiPS line.

In an embodiment, the endothelial cells are cultured in EGM-2 medium. Any other commercially available endothelial cell culture medium is suitable.

Detection of insulin producing cells can include for example detection of c-peptide positivity for example by flow cytometry and/or insulin and/or glucagon expression for example by RT-PCR.

In an embodiment, the insulin producing cells generated for example after co-culture with endothelial cells are monohormonal.

As used herein, the term "insulin producing cell" refers to a cell differentiated from a pancreatic progenitor which secretes insulin. An insulin producing cell includes functional pancreatic beta-cells, as well as pancreatic beta-like cells that synthesize, express, or secrete insulin in a constitutive or inducible manner. A population of insulin producing cells, e.g. produced by differentiating endodermal cells to pancreatic progenitors and subsequent differentiation into insulin producing cells according to the methods described herein, can be functional pancreatic beta-cells or beta-like cells (e.g., cells that have at least two characteristics of an endogenous functional beta-cell). It is also contemplated that the population of insulin producing cells, e.g. produced by the methods as disclosed herein, can comprise pancreatic beta-cells or pancreatic beta-like cells, and can also contain non-insulin producing cells (e.g. cells with a beta-cell like phenotype with the exception that they do not produce or secrete insulin).

The present disclosure also provides in another aspect endothelial cells useful to promote the maturation of NKX6-1⁺ pancreatic progenitors in vitro. It is useful to determine whether insulin-producing cells generated are functional or expected to be functional (e.g. if these insulin-producing cells co-express any other key pancreatic genes, such as PDX1 and NKX6-1). The method disclosed in the present application uses human endothelial cells (or components thereof), in co-culture with pancreatic progenitor cells and produces pancreatic lineage cells that co-express PDX1 and NKX6-1. The presently disclosed cells are expected to be functional. As shown in this disclosure, hESC-derived endothelial cells represent a suitable population for the induction and maturation of insulin-producing cells from hPSCs.

Accordingly in an embodiment, the methods induce the production of greater than about 10%, 20%, 25%, 30%, 35%, 40%, 45%, 50%, 55%, 60%, 65%, 70%, 75%, 80%, 85%, 90%, or about 95% NKX6-1 positive pancreatic progenitor cells from a population of endodermal cells and/or for example greater than about 5%, 10%, 15%, 20%, 25%, 30% 35%, 40%, 45%, 50%, 55%, 60%, 65%, 70%, 75%, 80%, 85%, 90%, or about 95% insulin producing cells from NKX6-1 positive pancreatic progenitor cells.

The cells can be harvested (e.g. spun down and resuspended in a suitable diluent) as a mixed cell culture.

In certain embodiments, the method further comprises enriching and/or isolating for example the NKX6-1 positive pancreatic progenitor cells and/or NKX6-1 positive pancreatic beta cells.

In an embodiment, the isolating step comprises contacting the population of cells with a specific agent that binds NKX6-1 positive cells.

It has also been demonstrated for example, in Example 4, that antibodies directed to HPx1 and HPx2 can be used to enrich for NKX6-1 positive cells. Specifically HPx1 and HPx2 antibodies are demonstrated to permit enrichment of NKX6-1 positive pancreatic progenitor cells.

Accordingly in an embodiment, the method comprises contacting a population of cells comprising NKX6-1 positive cells with an HPX epitope detecting antibody.

An "HPX epitope detecting antibody" as used herein, includes for example antibodies designated HPx1 and HPx2 available for example from Oregon Health&Science University (HPx2=OHSU#1038I), Beta Cell Biology Consortium (HPx2=AB2195 and HPx1=AB2194), Novus Biologicals (Hpx1=NBP1-18951, HPx2= NBP1-18952) as well as antibodies that recognize the same epitope as HPx1 or HPx2. For example, an antibody comprising the same CDR sequences as an HPx1 and/or HPx2 antibody, including for example humanized and chimeric forms, will detect the same epitope as HPx1 and/or HPx2.

It is also demonstrated herein that NKX6-1 positive progenitor cells express SOX9, PTF1a and SOX9, also known as SRY box 9 is a transcription factor; PTF1a, also known as a pancreas specific transcription factor 1a.

In an embodiment, the method comprises isolating/enriching for NKX6-1⁺ pancreatic progenitors comprising contacting a population of cells comprising pancreatic progenitor cells with an antibody to HPx1 and/or HPx2 and isolating or partially purifying the HPx1 and/or HPx2 bound subpopulation of cells.

In another embodiment, the method comprises isolating/enriching for NKX6-1⁺ pancreatic progenitors comprising contacting a population of cells comprising pancreatic progenitor cells with an antibody to HPx1 and/or HPx2 in combination with an antibody to CD142 isolating or partially purifying the HPx1 and/or HPx2 and CD142 bound subpopulation of cells.

In an embodiment, the HPx1 and/or HPx2 and/or CD142 antibody is conjugated to a bead such as a magnetic bead. In an embodiment, the enrichment comprises cell sorting for example by fluorescence activated cell sorting (FACS).

Example 4 shows for example that HPx stains approximately 60% of NENA induced NKX6-1 GFP positive cells at day 13.

The identification of two antibodies (HPx1 and HPx2) that stain the NKX6-1⁺ progenitor population will facilitate for example monitoring, quantifying and purifying the hPSC-derived pancreatic progenitor cells.

Prior to this disclosure, there was no indication or evidence that the antibodies HPx1 and HPx2 (referred to collectively as "HPx" herein) which were known to detect adult exocrine cells (Dorrell et al., 2008) recognize pancreatic progenitors. The use of these antibodies facilitates methods for identifying, monitoring, and isolating pancreatic progenitor cells derived for example from hESCs/hiPSCs or from other sources in a manner which preserves their viability for various downstream applications.

The HPx antibodies can be combined for example with other antibodies such as CD142 to optionally further increase the number of NKX6-1 positive cells in the enriched and/or isolated population.

Accordingly in an embodiment, the method comprises enriching and/or isolating a population of NKX6-1 positive pancreatic progenitor cells, wherein at least 30%, at least 35%, at least 40%, at least 45%, at least 50%, at least 55%, at least 60%, at least 70%, at least 75%, at least 80%, at least 85%, at least 90%, at least 95% or greater of the total cells in the isolated population are NKX6-1 positive pancreatic progenitor cells.

The isolating/enriching methods can also include for example using other agents such as antibodies to other pancreatic progenitor surface markers such as CD142.

In an embodiment, the population of cells comprising NKX6-1 positive cells is a NKX6-1 positive population produced according to a method described herein.

The term "isolated population of cells" as used herein refers to a population of cells that has been removed and separated from a mixed or heterogeneous population of cells. In some embodiments, an isolated population is a substantially pure population of cells as compared to the heterogeneous population from which the cells were isolated or enriched from.

The term "substantially pure", with respect to a particular cell population, refers to a population of cells that is at least about 65%, preferably at least about 75%, at least about 85%, more preferably at least about 90%, and most preferably at least about 95% pure, with respect to the cells making up a total cell population. Similarly, with regard to a "substantially pure" population of NKX6-1-positive pancreatic progenitors, refers to a population of cells that contain fewer than about 30%, fewer than about 20%, more preferably fewer than about 15%, 10%, 8%, 7%, most preferably fewer than about 5%, 4%, 3%, 2%, 1%, or less than 1%, of cells that are not NKX6-1-positive pancreatic progenitors or their progeny as defined by the terms herein. In some embodiments, the present invention encompasses methods to expand a population of NKX6-1-positive pancreatic progenitors, wherein the expanded population of NKX6-1-positive pancreatic progenitors is a substantially pure population of NKX6-1-positive pancreatic progenitors.

The terms "enriching" or "enriched" are used interchangeably herein and mean that the yield (fraction) of cells of one type is increased by at least about 10%, at least about 20%, at least about 30%, at least about 40%, at least about 50% or at least about 60% over the fraction of cells of that type in the starting culture or preparation. Enriching and partially purifying can be used interchangeably.

### Compositions and Kits

As discussed above NKX6-1 pancreatic progenitor cells and differentiated insulin producing cells can be isolated. Accordingly a further aspect described herein includes an enriched, purified or isolated cell population of NKX6-1 positive pancreatic progenitor cells and/or differentiated insulin producing cells, for example produced according to a method described herein. Another aspect described includes a composition comprising an enriched, purified or isolated cell population of NKX6-1 positive pancreatic progenitor cells and/or differentiated insulin producing cells; and a suitable diluent.

A suitable diluent includes for example a suitable culture medium, or freezing medium containing for example serum, a serum substitute or serum supplement and/or a suitable cryoprotectant such as dimethyl sulphoxide (DMSO), glycerol Methylcellulose or polyvinyl pyrrolidone.

A further described aspect comprises a culture medium supplement composition comprising an EGF component, a Nicotinamide component and/or a Noggin component, which can be used as a supplement for a cell culture base medium. The supplement can also include other components discussed herein such as ascorbic acid, L-glutamine and B27. The supplement in an embodiment comprises the active ingredients: Noggin, EGF, Nicotinamide, (e.g. NENA supplement) and optionally one or more of Ascorbic Acid, L-Glutamine and B27. In other embodiments the supplement includes for example an Exendin-4 component (e.g NENAEx supplement).

The components for differentiating NKX6-1+ cells from endodermal cells can be comprised as a single supplement to be added to base media such DMEM (or for example IMDM, RPMI, CMRL). The supplement would contain in an embodiment the active ingredients: Noggin, EGF, Nicotinamide, Ascorbic Acid, L-Glutamine and B27.

A further described aspect is a culture medium composition suitable for differentiating cells from one stage to another. For example, a suitable Stage 4 culture medium composition can comprise for example an EGF component, and/or a Nicotinamide component, optionally a Noggin component, and/or additionally other growth factors etc. described herein optionally a Exendin-4 component, for example that can promote Stage 4 differentiation.

The term "cell culture medium" (also referred to herein as a "culture medium" or "medium") as referred to herein is a medium for culturing cells containing nutrients that maintain cell viability and support proliferation and optionally differentiation. The cell culture medium may contain any of the following in an appropriate combination: salt(s), buffer(s), amino acids, glucose or other sugar(s), antibiotics, serum or serum replacement, and other components such as peptide growth factors, vitamins etc. Cell culture media ordinarily used for particular cell types are known to those skilled in the art.

The suitable culture medium can include a suitable base culture medium including for example DMEM (Life Technologies), IMDM, RPMI, CMRL and/or any other or media that supports the growth of endodermal cells to provide for example a base culture medium composition to which components and optionally other agents can be added (e.g. to provide a suitable Stage 4 culture medium composition).

Stage specific culture mediums can be prepared, for example a Stage 4 culture medium (e.g. NENA or NENAEx medium). The culture medium can for example be used can to culture the population of endoderm cells to induce NKX6-1 positive differentiation.

Accordingly a further aspect described herein is a culture medium composition comprising:
a cell culture base medium; and
one or more of:
   i) an EGF component,
   ii) a Nicotinamide component, and
   iii) a Noggin component and
   iv) optionally an Exendin-4 component.

The medium composition (or base medium) can further comprise one or more antibiotics such as penicillin and streptomycin; one or more amino acids such as L-Glutamine (Life Technologies), the serum substitute B27 Supplement (Life Technologies) and/or one or more vitamins such as L-ascorbic acid (SIGMA). A suitable recipe is provided for example in Example 8.

The amount of the components in the supplement can for example be amounts that when diluted in a culture medium (e.g. when diluted in a 450 mL base medium) result in concentrations described herein for differentiating for example an endodermal population. Similarly, the concentration of the components in the culture medium can be concentrations described herein for differentiating for example an endodermal population.

A further aspect described herein includes a kit comprising:
an EGF component,
a Nicotinamide component, and/or
a Noggin component and/or
optionally an Exendin-4 component.

As described above, the composition and kit components can include any of the components described elsewhere herein and optionally instructions for use. For example, in an embodiment, the kit comprises a supplement comprising components etc. to induce differentiation of one or more stages described herein (e.g. Stage 4 supplement). In an embodiment, the kit comprises a base culture medium, optionally a base culture medium described herein.

### Uses

The NKX6-1+ HPSC-derived pancreatic progenitors (e.g. HPx1/2 enriched) and their beta cells derivatives could be used for a number of applications.

For example, the NKX6-1 pancreatic progenitor cells can be used for predictive drug toxicology and drug discovery.

Accordingly, in an embodiment is provided is an assay comprising: contacting NKX6-1-expressing population generated using a method described herein with a test compound, and determining if the test compound: 1)) stimulates expansion, 2) triggers apoptosis, and/or 3) induces differentiation of the NKX6-1 expressing cells to insulin producing beta-like cells to other hormone-producing cells (e.g. somatostatin, glucagon, pancreatic polypeptide and ghrelin), acinar or ductal cells; compared to a control. Assays known in the art can be used for example to assess apoptosis and/or cell expansion. Differentiation to hormone producing cells can be assessed by measuring hormone production, optionally secreted hormone or by assessing mRNA levels.

The use of an HPX antibody can facilitate the compound screening, by providing a more homogenous population for contacting with the test compound. Additionally, the population enriched for insulin+ cells generated by co-culture with endothelial cells can be used to test for compounds that can trigger proliferation or apoptosis and for compounds that can improve glucose-stimulated insulin secretion.

The cells described can also be used for cell transplantation as shown for example in Example 3. For example, mixed population of cells, enriched and/or isolated NKX6-1 positive pancreatic progenitor cells and/or NKX6-1 positive insulin producing cells can be introduced into a subject in need thereof, for example for treating diabetes and/or a pre-diabetes condition.

Accordingly, an aspect includes obtaining cells and/or preparing pancreatic precursor cells and/or insulin producing cells according to a method described herein, and administering said cells to a subject in need thereof, for example a subject with diabetes.

Also included are uses of said cells and compositions comprising said cells for transplanting and/or treating a subject in need thereof, for example for transplanting and/or treating a subject with diabetes.

In an embodiment, the diabetes is type I diabetes. In another embodiment, the diabetes is type II diabetes.

In an embodiment the NKX6-1 positive pancreatic progenitor cells and/or beta cells are used in tissue engineering. For example, access to purified populations of pancreatic lineage cells will enable the generation of engineered constructs with defined numbers of pancreatic cells.

In an embodiment, the methods are applied to patient specific disease hiPSCs and used for example to model diabetes. For example, pancreatic or other cells from a patient with diabetes can be isolated, treated to obtain hiPSCs which can then be cultured and induced to differentiate to NKX6-1 pancreatic progenitor cells. These cells can be used to assess characteristics of the disease, such as the genes involved in the disease or the response to patients' immune cells.

For example, normal cells and patient specific disease hiPSCs can induced to NKX6-1 positive pancreatic and endothelial cells and compared. For example, genetic, epigenetic and proteomic analyses of pancreatic progenitors and beta cells from normal and patient specific hiPSCs can be conducted. Such detailed analyses can lead to the discovery of signaling pathways, transcriptional regulatory networks and/or cell surface markers that regulate normal human pancreatic development as well as those that play a role in disease.

The term "subject" as used herein includes all members of the animal kingdom including mammals, and suitably refers to humans.

The terms "treat", "treating", "treatment", etc., as applied to an isolated cell, include subjecting the cell to any kind of process or condition or performing any kind of manipulation or procedure on the cell. As applied to a subject, the terms refer to providing medical or surgical attention, care, or management to an individual.

The term "treatment" as used herein as applied to a subject, refers to an approach aimed at obtaining beneficial or desired results, including clinical results and includes medical procedures and applications including for example pharmaceutical interventions, surgery, radiotherapy and naturopathic interventions as well as test treatments for treating diabetes. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

As used herein, the terms "administering," "introducing" and "transplanting" are used interchangeably in the context of delivering cells (e.g. NKX6-1 positive pancreatic progenitor cells, or their differentiated progeny (e.g. insulin-producing cells such as pancreatic .beta.-cells) into a subject, by a method or route which results in at least partial localization of the introduced cells at a desired site. The cells can be implanted directly to the pancreas, or alternatively be administered by any appropriate route which results in delivery to a desired location in the subject where at least a portion of the implanted cells or components of the cells remain viable.

Further, the definitions and embodiments described in particular sections are intended to be applicable to other embodiments herein described for which they are suitable as would be understood by a person skilled in the art. For example, in the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The above disclosure generally describes the present application. A more complete understanding can be obtained by reference to the following specific examples. These examples are described solely for the purpose of illustration and are not intended to limit the scope of the application.

The following non-limiting examples are illustrative of the present disclosure:

### Examples

### Example 1

### Noggin, EGF, Nicotinamide and Exendin-4 induce NKX6-1 expression in hESC-derived endodermal cells.

Pancreatic progenitor cells with the potential to generate functional beta cells are characterized by the expression of key transcription factors: PDX1, SOX9, NKX6-1 and PTF1A. NKX6-NKX6-11 is of particular significance, as it is essential for the development of a functional beta cell population (Sander et al., 2000). To date, the factors regulating the development of these progenitors from PDX1 pancreatic endoderm are not known.

A robust method is described herein to generate NKX6-1⁺ endodermal cells from hPSCs with potential to generate functional beta cells. Using a reference hESC-reporter cell line in which GFP has been targeted to the NKX6-1 locus by homologous recombination, factors that can induce the development of a NKX6-1-GFP⁺ population were identified. With this approach, hPSCs are differentiated to the "pancreatic bud stage" (PDX1⁺-expressing cells) over a 7 day period, using a previously published protocol (Nostro et al., 2011). These cells are then cultured in one or more components optionally a combination of the BMP inhibitor (Noggin or other BMP antagonists), EGF (or EGF-related factors), Nicotinamide and Exendin-4 (NENAEx) (Figure 1) and analysed at different times for expression of NKX6-1-GFP. NKX6-1-GFP expression, as measured by flow cytometry was detected within 3 days of culture in NENAEx (Figure 2).

Figure 1 schematically illustrates that HPSCs are differentiated up to stage 3 (d7) according to a previously described protocol (Nostro et al., 2011), with slight modifications (addition of Wnt3a at day 1 and Exendin-4, Ex-4 from day 5 to day 7). Cytokines and small molecules were used at the following concentrations: Activin A (Act1: 100 ng/ml), Wnt3a (Stage 1: 25 ng/ml), bFGF (5 ng/ml), FGF10 (50 ng/ml), Wnt3a (Stage 2: 3 ng/ml), Dorsomorphin (DM: 0.75 µM), Noggin (NOG: 50 ng/ml), Cyclopamine-KAAD (Cyc: 2.5 µM), Retinoic Acid (RA: 2 µM) Exendin-4 (Ex-4: 50 ng/ml) EGF (50 ng/ml), Nicotinamide (NA: 10 mM).

Figure 2 demonstrates that Noggin, EGF, Nicotinamide and Exendin-4 induce NKX6-1:GFP expression in hESC-derived endodermal cells. Kinetic analysis for GFP (day 7 to day 17, T7-T17) during pancreatic differentiation of NKX6-1^{GFP/w} hESC line. NKX6-1^{GFP/w} cells were differentiated according to published protocol (Nostro et al., 2011) up to stage 3 and then treated with Noggin (50 ng/ml), EGF (50 ng/ml), Nicotinamide (10 mM) and Exendin-4 (50 ng/ml) for 11 days. GFP, as measured by flow cytometry, starts to be detected at day 10 and reaches maximal expression at day 13 of differentiation. Error bars represent standard error of the mean (n=3).

The proportion of NKX6-1-GFP+ cells increased dramatically over the following 3 days (e.g. days 11-13 in culture),, representing greater than 60% of the entire culture by day 13. The levels of NKX6-1-GFP remained constant for the next 2 days and then declined modestly by day 17 of culture.

Molecular analyses (RT-qPCR) revealed an upregulation of NKX6-1 expression between days 9 and 11, follow by a steady decline to day 17(Figure 3).

Figure 3 shows that NKX6-1 induction coincides with PDX1, PTF1A and SOX9 expression. QPCR analysis for NKX6-1, PDX1, PTF1A and SOX9 during Kinetic analysis for GFP (day 7 to day 17, T7-T17) during pancreatic differentiation of NKX6-1^{GFP/w} hESC line. NKX6-1^{GFP/w} cells were differentiated according to published protocol (Nostro et al., 2011) up to stage 3 and then treated with Noggin (50 ng/ml), EGF (50 ng/ml), Nicotinamide (10 mM) and Exendin-4 (50 ng/ml) for 11 days. Expression levels are normalized to the housekeeping gene TBP and compared to adult pancreas (AP, grey bar) expression levels. Error bars represent standard error of the mean (n=2).

The difference in kinetics of mRNA and GFP expression may reflect a delay in the transgene expression and differences in the stability of the NKX6-1 message and GFP protein. Expression of PDX1 was upregulated several days earlier, between days 7 and 8 (Figure 3). The levels of PDX1 expression peaked at day 10 and then declined over the next 7 days. The expression pattern of PTF1A was transient and detected between 10 and 14 of differentiation. SOX9 expression was upregulated between days 8 and 10 and then remained relatively constant throughout the duration of the experiment. Taken together, these findings demonstrate that the combination of NENAEx induces the development of endocrine progenitors from pancreatic endoderm.

### Example 2

### The combination of Noggin, EGF and Nicotinamide (NENA) induces NKX6-1 expression in hESC-derived endodermal cells.

To further determine which of the combination of NENAEx factors was essential for the generation of the NKX6-1⁺ progenitors, different combinations on the reporter NKX6-1-GFP hESCs as well as on a second unmanipulated hESC line, reference H1 (WA01) were tested. Intracellular flow cytometry was used to evaluate the proportion of NKX6-1⁺ cells in the H1-derived populations. As shown in Figure 4, the combination of Noggin, EGF, and Nicotinamide (NENA) was as effective as the 4 factor combination of Noggin, EGF, Nicotinamide and Exendin-4 at inducing NKX6-1. The combination of EGF and Nicotinamide (ENA) did induce NKX6-1 expression (46.9±7.4%), however the levels were always lower compared to the combination of the 3 factors (NENA) (Figure 4A). H1 (WA01) differentiation showed a very similar pattern in response to NENA treatment (Figure 4B). Similar data has been obtained using the reference H9 cell line, demonstrating that this induction protocol is not cell line dependent and is widely applicable to the generation of hPSC-derived NKX6-1 progenitors.

**Figure 4A** demonstrates that using the NKX6-1^{*GFP*/*w*} HESC line, the percentage of NKX6-1:GFP+ cells is highest when endodermal cells are cultured in the presence of Noggin, EGF, Nicotinamide (with or without Exendin-4), compared to no treatment or various combination of the above compounds.

**Figure 4B** is a graph that demonstrates that using the reference H1 (WA01) HESC line, the percentage of NKX6-1+ cells is highest when endodermal cells are cultured in the presence of Noggin, EGF, Nicotinamide (with or without Exendin-4), compared to no treatment or various combination of the above compounds.

### Example 3

### NKX6-1 cells generated using the "NENA treatment" have the potential to generate insulin-producing cells.

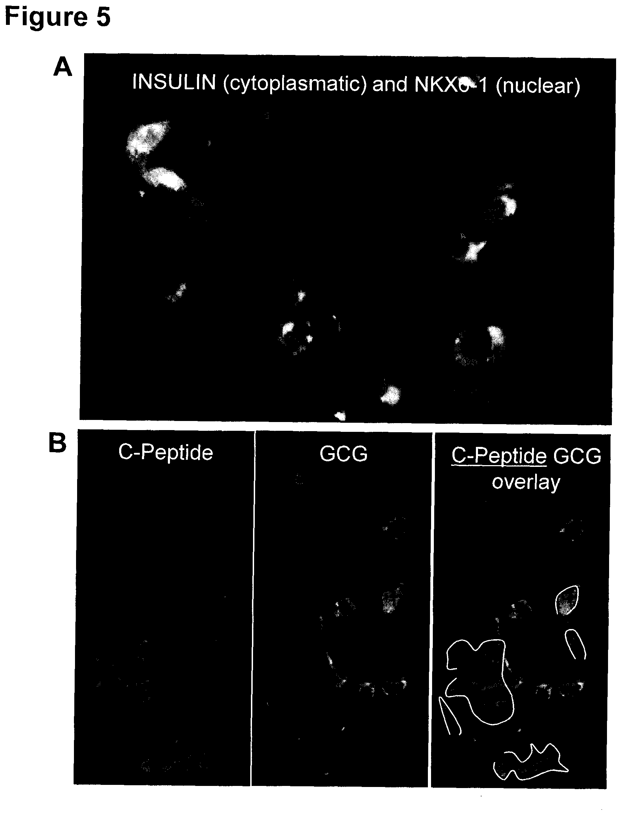

To establish the developmental potential of the hPSC-derived NKX6-1-enriched population, 5-10x10⁶ NENA-induced cells were transplanted (day 14 of differentiation) in the mammary fat pad of immunocompromised mice. Graft analysis at 4 weeks post-transplantation revealed the presence of ductal-like structures (e.g. CK19+) that maintained expression of both PDX1 and NKX6-1. At this stage the grafts contained few glucagon⁺ cells and almost no insulin⁺ cells. At 6 weeks post transplant, the grafts contained substantial numbers of NKX6-1⁺ cells (Figure 5A). The 6 week grafts contained a higher percentage of insulin⁺ cells compared to the 4 week grafts. These insulin⁺ cells all co-expressed NKX6-1 (Figure 5A). To demonstrate that the insulin⁺ cells present in the graft represent hPSC-derived insulin-producing cells, immunohistochemistry was performed using an anti-human-c-peptide antibody. As shown in Figure 5B, the graft did contain c-peptide+ cells, confirming that insulin is being produced by the human cells and not up taken from the blood. Additionally, co-staining with an anti-glucagon antibody revealed that the c-peptide⁺ cells do not express glucagon, indicating that they are not polyhormonal (Figure 5B). The findings from these transplantation studies demonstrate that NENA-induced progenitors are able to differentiate to insulin producing cells and ductal cells following transplantation in vivo.

### Example 4

### HPx antibodies mark NKX6-1+ progenitors.

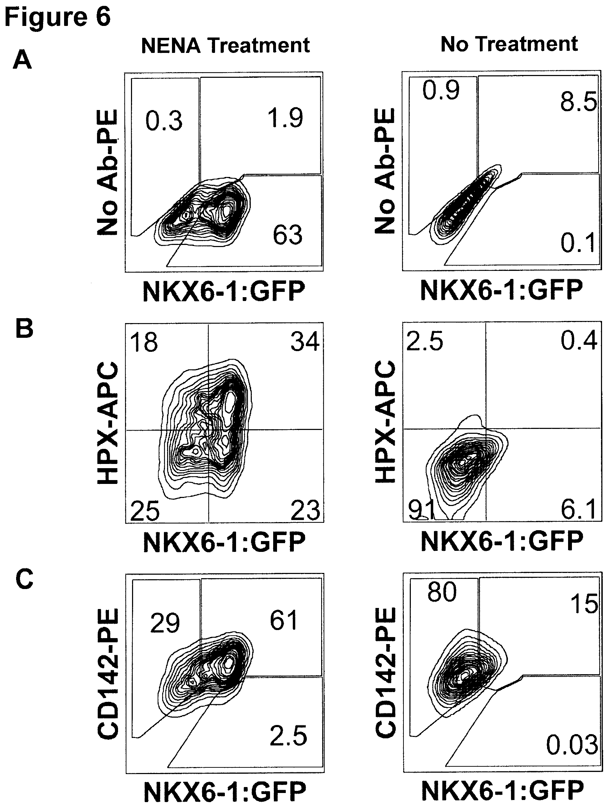

Recent studies identified the surface marker CD142 as a potential epitope that could be used to isolate multipotent pancreatic progenitors from hPSC-derived cultures (Kelly et al., 2011). Through the analyses of a library of different antibodies, two previously described antibodies HPx1 and HPx2 were found to stain a portion of the NKX6-1+ progenitor population. As shown in Figure 6, HPx (e.g. HPx1 and/or HPx2) stains approximately 60% of NENA-induced NKX6-1-GFP⁺ population at day 13 of culture. HPx also stains a population of NKX6-1-GFP⁻ negative cells. Very few HPx⁺ cells were detected in non-treated cultures, demonstrating that HPx staining correlates well with the emergence of NKX6-1⁺ progenitors. The anti-CD142 antibody marks a significant portion of the NKX6-1-GFP population as reported by Kelly et al., (Kelly et al., 2011) (Figure 6C). However, the non-induced cultures also contain a large CD142 population, in spite of the fact few NKX6-1⁺ cells are detected under these conditions (Figure 6C). These findings indicate that expression of CD142 does not correlate well with the development of NKX6-1⁺ progenitor. The patterns of HPx staining suggests that it will be a superior reagent for monitoring the development of NKX6-1⁺ progenitors and for isolating them from mixed cell cultures.

### Example 5

### HPx antibodies can be used to purify a population enriched for NKX6-1 positive cells.

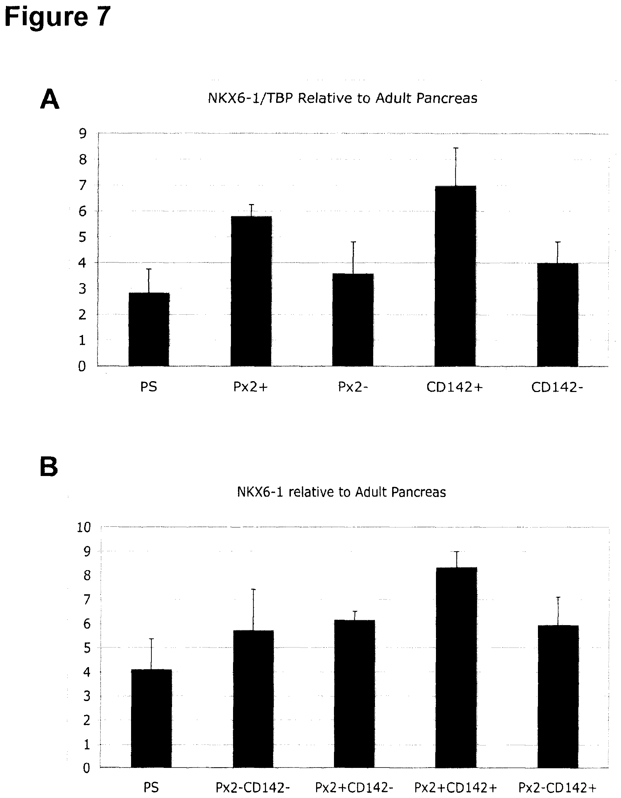

Two experiments were run comparing HPx (e.g. HPx, HPx1, HPx2, Px, Px1 and Px2 are used interchangeably: HPx1 and HPx2 recognize the same population and HPx 1 and HPx2 can be used interchangeably, and/or together) vs CD142 antibodies (Figure 7).

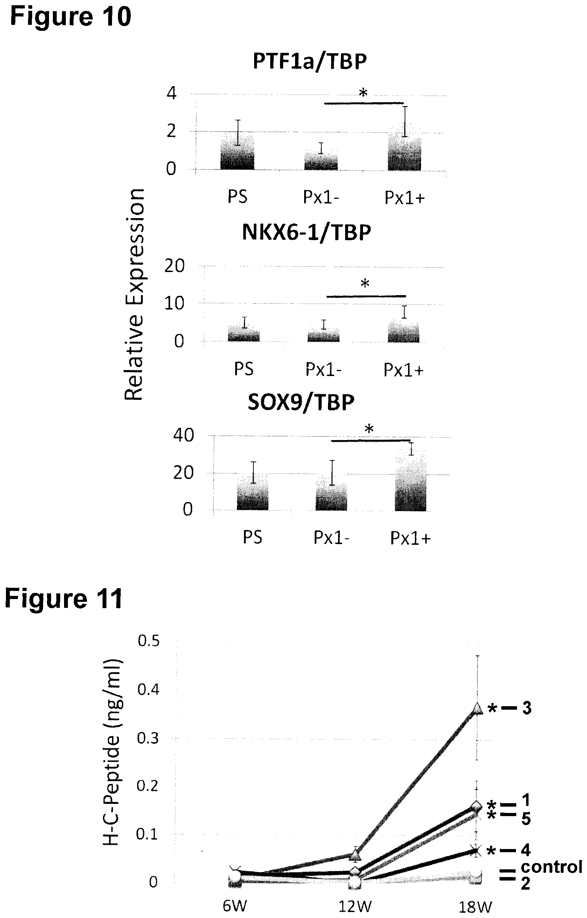

Cells positive and negative for the two antibodies were isolated from d14 NENA-differentiated cells and the fraction that expressed the highest levels of NKX6-1 was determined (Figure 7A and 10).

These results suggest that HPx can be used to enrich for NKX6-1 positive cells. CD142+ and Px2+ fraction showed a 2-fold increase in the expression levels of NKX6-1 compared to the pre-sort (PS) population. While both antibodies can be used to enrich for NKX6-1 positive cells, HPx shows higher specificity (see Figure 6C) and provides higher cell yield (approximately 3X more cells can be isolated using HPx compared to CD142 in the same time frame).

The two antibodies (CD142 and HPx) were also used in combination to sort Px2+ and CD142+ cells from d14 NENA-differentiated cells and the expression levels of NKX6-1 was evaluated in the different fractions: pre-sort (PS), Px2-CD142-, Px2+CD142-, Px2+CD142+, Px2-CD142+ sorted populations (Figure 7B). NKX6-1 was expressed at the highest levels in the Px2+CD142+ double positive population, however, the fold increase over PS was the same as the single CD142+ or single Px2+ population, suggesting that there is little advantage into double sorting.

Figure 7 shows quantitative PCR (QPCR) analysis for NKX6-1 in the pre-sort (PS), Px2+, Px2-, CD142+, CD142- sorted populations from a day 14 NENA-differentiated H1 (WA01) cells. (B) Quantitative PCR (QPCR) analysis for NKX6-1 in the pre-sort (PS), Px2-CD142-, Px2+CD142-, Px2+CD142+, Px2-CD142+ sorted populations from a day 14 NENA-differentiated H1 (WA01) cells. Error bars represent standard error of the mean (n=2 for A and n=3 for B). NKX6-1 expression is normalized to a housekeeping gene TBP (TATA-Binding Protein).

### Example 6

### Generation of endothelial cells capable of promoting maturation of c-peptide⁺ cells.

HES-derived endothelial cells were generated using the reference H1 hESC cell line by induction with the combination of BMP4, bFGF, VEGF. At day 6, CD34⁺ were sorted and cultured in EGM-2 media (Lonza) supplemented with 50 ng/ml bFGF and 100 ng/ml VEGF. Analyses of the day 6 CD34⁺ cells revealed that they express KDR, CD31 and vWF and display the capacity to take up acetylated LDL (Fig. 8).

Any endothelial population can be used particularly endothelial cells derived from embryonic stem cells.

### Example 7

### Co-culture of hESC-derived endothelial cells and pancreatic progenitors increases the pool of NKX6-1+ progenitors and promote their maturation into insulin producing cells.

For these studies, NENAEx-induced progenitors at day 10 of culture generated from the NKX6-1-GFP reporter cell line were aggregated and the aggregates seeded onto confluent hESC-derived CD34+ endothelial cells. These cultures were maintained for an additional 7 days in NENAEx-containing media with 5ng/ml bFGF and 100ng/ml VEGF. As controls, cells were kept as monolayer or aggregates were cultured on collagen-coated dishes in the absence of endothelial cells. As shown in Figure 9A, cells cultured on the endothelial cells maintained a higher proportion of NKX6-1-GFP+ cells than those cultured as monolayer or as aggregates on collagen. Analyses of the different populations for the presence of insulin producing cells revealed striking differences. Only the aggregates co-cultured with the endothelial cells generated substantial numbers of c-peptide+ cells. Approximately 33% of the NKX6-1-GFP+ cells were c-peptide+, representing 17% of the entire aggregate population (33% of the 53% GFP cells). RT-qPCR analyses confirmed the flow cytometric analyses and showed that only the population co-cultured with the endothelial cells expressed both insulin and glucagon message (Figure 9B). These findings demonstrate that hESC-derived endothelial cells are able to support the maturation of c-peptide+ cells from NKX6-1+ progenitors. The fact that the c-peptide+ cells co-express NKX6-1-GFP strongly suggests that they are monohormonal beta cells.

### Example 8

**Table 1. Exemplary formula for Stage 4 MEDIUM composition**

| | Stock Conc. | FinalConc. | per mL |
|---|---|---|---|
| DMEM (Life Technologies)* | | | 1 mL |
| L-Glutamine (Life Technologies) | 100x | 1% | 10 µL |
| B27 Supplement (Life Technologies) | 100x | 1% | 10 µL |
| L-ASCORBIC ACID (AA) (SIGMA) | 5 mg/mL | 50 µg/mL | 10 µL |
| Noggin (R&D Systems) | 100 µg/mL | 50 ng/mL | 0.5 µL |
| EGF (R&D Systems) | 100 µg/mL | 50 ng/mL | 0.5 µL |
| Nicotinamide (SIGMA) | 1.64 M | 10 mM | 6 µL |
| Exendin-4** (SIGMA) | 50 µg/ml | 50 ng/mL | 1 µL |

| | | | |
|---|---|---|---|
| * DMEM could be substituted by other base-media such as IMDM, RPMI, CMRL, or any other media that supports the growth of endodermal cells. Base media contains antibiotics: Penicillin/Streptomycin (Life Technologies). **Exendin-4 is not required for NKX6-1 induction. | | | |

Co-culture with endothelial cells to generate insulin+ cells is supported by the addition of bFGF and VEGF (R&D Systems).

### Example 9

**Markers of the Posterior Foregut (Stage 3) population.** Endodermal cells at stage 3 must express at least FOXA2 and can be positive or negative for PDX1 (other factors such as HNF1β, hepatocyte nuclear factor 4, alpha (HNF4A) **Gene ID: 3172** and one cut homeobox 1 (ONECUT1/HNF6) **Gene ID: 3175** can for example also be expressed).

**Markers of the Pancreatic Progenitor (Stage 4) population.** Cells differentiated into NKX6-1+ pancreatic progenitors can in addition to expressing NKX6-1, also express FOXA2, PDX1, SRY (sex determining region Y)-box 9 (SOX9) **Gene ID 6662** and pancreas specific transcription factor, 1a (PTF1A) **Gene ID:256297.**

### Example 10

### Examples of Noggin components:

- Noggin
- BMPR1A
- BMPR1B
- Dorsomorphin
- LDN 193189
- CHORDIN
- Or combinations of the above

### Example 11

### Examples of EGF components:

- EGF
- Amphiregulin (AR)
- Transforming Growth Factor α (TGFα)
- Betacellulin (BTC)
- Heparin-binding EGF (HB-EGF)
- Epiregulin (EPR)
- Neuregulins (NRG1. NRG2, 3 NRG4)
Or combinations of the above and EGF Factors that can impact downstream signaling molecules including for example MAPK or PI3-kinase activators

### Example 12

### Examples of Nicotinamide Components:

- Poly(ADP-ribose) synthetase inhibitors for example benzamide and 3-aminobenzamide (Otonkoski et al., 1993)
- Sir-2 inhibitors for example sirtinol, M15, splitomicin (Bitterman et al., 2002).
- Other HDAC inhibitors including for example AN-9, CI-994, FK228, LAQ-824, MS275, SAHA, Valproic Acid, TSA, Sodium Butyrate (Bitterman et al., 2002).

### Example 13

**Table 2 Percentage of NKX6-1+ cells at day 13 of differentiation using the human ES cell line: H9 (WA09)**

| **Treatment at stage 4** | **Percentage of NKX6-1+ cells** |
|---|---|
| No Treatment | 6.1 |
| Noggin (N) | 21.7 |
| EGF (E) | 18.5 |
| Nicotinamide (NA) | 14.0 |
| Noggin EGF (NE) | 16.6 |
| Noggin Nicotinamide (NNA) | 12.6 |
| EGF Nicotinamide (ENA) | 38.3 |
| Noggin EGF Nicotinamide (NENA) | 56.4 |
| Noggin Exendin-4 EGF Nicotinamide (NExENA) n=1 | 34.86 |

### Example 14

HPSC-derived pancreatic progenitors (HPx1/2 enriched) and their beta cells derivatives could be used for predictive drug toxicology and drug discovery.

Specifically, the NKX6-1-expressing population generated using the culture conditions described herein can be used to identify compounds that can: 1) stimulate expansion, 2) trigger apoptosis, 3) induce differentiation of the NKX6-1 expressing cells to beta-like cells as well as to other hormone-producing cells (somatostatin, glucagon, pancreatic polypeptide and ghrelin), acinar or ductal cells. The use of the HPX antibody can facilitate the compound screening, by providing a homogenous population. Additionally, the population enriched for insulin+ cells generated by co-culture with endothelial cells can be used to test for compounds that can trigger proliferation or apoptosis and for compounds that can improve glucose-stimulated insulin secretion.

### Example 15

1) Cell transplantation into diabetic animal models to test the effectiveness and functionality of the NKX6-1-positive and/or insulin-positive cells generated in vitro. Briefly cells will be transplanted in different immunodeficient animal models where diabetes can be induced genetically or by drug treatment (such as streptozotocin treatment) and the functionality of the human-PSC-derived pancreatic cells (NKX6-1-positive and/or insulin-positive) will be assessed by measuring blood glucose concentration and human C-peptide levels in the serum of the animal.
2) Cell transplantation: a) the effects of transplanting progenitor populations vs functional beta cells are compared, b) the effects of transplanting HPx1/2-enriched pancreatic progenitors vs mixed lineage populations consisting of defined numbers of pancreatic cells and other cell types including endothelial cells and fibroblasts are compared.

### Example 16

Pancreatic progenitor cells were prepared as described in Example 1 using NENA. As shown in Figure 10, HPx1+ cells were isolated using fluorescence activated cell sorting (FACS) and are enriched for a population that expresses higher levels of NKX6-1/TBP, SOX9/TBP, and PTF1a/TBP mRNA compared to HPx1- cells and presorted cells (PS).

### Example 17

NKX6-1+ pancreatic cells were generated using the "NENA" treatment described in Example 1 and were transplanted into immunocompromised mice. These cells generate functional insulin producing cells as indicted by human C-peptide secretion in response to glucose challenge. Human C-peptide secretion levels in response to glucose challenge was measured by ELISA at 6, 12 and 18 weeks post-transplantation of H1 (WA01) d13 differentiated cells into immune-compromised mice (Figure 11). Five transplantations (identified as 1, 2, 3, 4, and 5) plus a transplantation using control cells (identified as "control") were performed. Mice 1, 3, 4 and 5 had significantly elevated levels of serum C-peptide.

### CITATIONS FOR REFERENCES REFERRED TO IN THE SPECIFICATION

Bitterman, K.J., Anderson, R.M., Cohen, H.Y., Latorre-Esteves, M. and Sinclair, D.A. 2002 Inhibition of Silencing and Accelerated Aging by Nicotinamide, a putative negative regulator of yest Sir2 and Human SIRT1. JBC 277(47): 45099
Dorrell, C., Abraham, S.L., Lanxon-Cookson, K.M., Canaday, P.S., Streeter, P.R., and Grompe, M. 2008. Isolation of major pancreatic cell types and long-term culture-initiating cells using novel human surface markers. Stem Cell Res 1(3): 183-194.
Jaramillo, M. and Banerjee, I. 2012. Endothelial Cell Co-culture Mediates Maturation of Human Embryonic Stem Cell to Pancreatic Insulin Producing Cells in a Directed Differentiation Approach. J Vis Exp(61).
Kelly, O.G., Chan, M.Y., Martinson, L.A., Kadoya, K., Ostertag, T.M., Ross, K.G., Richardson, M., Carpenter, M.K., D'Amour, K.A., Kroon, E., Moorman, M., Baetge, E.E., and Bang, A.G. 2011. Cell-surface markers for the isolation of pancreatic cell types derived from human embryonic stem cells. Nat Biotechnol 29(8): 750-756.
Lammert, E., Cleaver, O., and Melton, D. 2001. Induction of pancreatic differentiation by signals from blood vessels. Science 294(5542): 564-567.
Nostro, M.C., Sarangi, F., Ogawa, S., Holtzinger, A., Corneo, B., Li, X., Micallef, S.J., Park, I.H., Basford, C., Wheeler, M.B., Daley, G.Q., Elefanty, A.G., Stanley, E.G., and Keller, G. 2011. Stage-specific signaling through TGFbeta family members and WNT regulates patterning and pancreatic specification of human pluripotent stem cells. Development 138(5): 861-871.
Otonkoski, T., Beattie, G.M., Mally, M.I., Ricordi, C., and Hayek, A. 1993. Nicotinamide is a potent inducer of endocrine differentiation in cultured human fetal pancreatic cells. J Clin Invest 92(3): 1459-1466.
Sander, M., Sussel, L., Conners, J., Scheel, D., Kalamaras, J., Dela Cruz, F., Schwitzgebel, V., Hayes-Jordan, A., and German, M. 2000. Homeobox gene NKX6-1 lies downstream of Nkx2.2 in the major pathway of beta-cell formation in the pancreas. Development 127(24): 5533-5540.
Yoshitomi, H. and Zaret, K.S. 2004. Endothelial cell interactions initiate dorsal pancreas development by selectively inducing the transcription factor Ptf1a. Development 131(4): 807-817.

## Claims

1. A method of producing NKX6-1+ pancreatic progenitor cells from an endodermal cell population, the method comprising contacting the endodermal cell population with a combination of 1) at least one EGF component selected from EGF, an active conjugate of EGF and an active fragment of EGF; and 2) nicotinamide or a salt thereof to induce the differentiation of at least a portion of the endodermal cell population into NKX6-1+ pancreatic progenitor cells.

2. The method of claim 1, wherein the combination further comprises at least one noggin component selected from Noggin, an active conjugate of Noggin, an active fragment of Noggin, BMPR1A, BMPR1B, Dorsomorphin, LDN193189, chordin and any combination of two or more thereof.

3. The method of claim 1 or 2, wherein the EGF component is EGF or Heparin-binding EGF (HB-EGF).

4. The method of claim 2 or 3, wherein the combination comprises Noggin, EGF and nicotinamide.

5. The method of any one of claims 1 to 4, wherein the combination further comprises Exendin-4 or an active fragment thereof.

6. The method of any one of claims 1 to 5, wherein the endodermal cell population is differentiated from pluripotent stem cells (PSCs) preferably an embryonic stem cell (ESC) or an induced pluripotent stem cell (iPSCs).

7. The method of claim 6, wherein the pluripotent stem cell is a human ESC (hESC) or a human iPSC (hiPSC).

8. The method of any one of claims 1 to 7, first comprising producing an endodermal cell population.

9. The method of claim 8, wherein the method of producing the endodermal cell population comprises one or more of steps or substeps a, b and c; and step d, the steps comprising:
a) contacting a pluripotent stem cell population with a combination of:
I) a nodal agonist, preferably ActA and a wnt signaling agonist, preferably, Wnt3a or CHIR 99021,
II) a nodal agonist, preferably Act A, a FGF agonist, preferably bFGF, and
III) a nodal agonist, preferably ActA and a FGF agonist, preferably bFGF;
to produce Stage 1 differentiated cells;
b) contacting the Stage I differentiated cells with a FGF agonist, preferably FGF10, and a noggin component, preferably Dorsomorphin, to produce Stage 2 differentiated cells;
c) contacting the Stage 2 differentiated cells with a Noggin, Retinoic acid (RA) or RA analog, a hedge-hog signaling inhibitor preferably cyclopamine-KAAD (Cyc), a FGF agonist, preferably FGF10 and Exendin-4 to provide an endodermal cell population; and
d) contacting the endodermal cell population with a combination comprising at least one EGF component selected from EGF, an active conjugate of EGF and an active fragment of EGF and nicotinamide or a salt thereof in an amount sufficient to induce the differentiation of at least a portion of the endodermal cell population into NKX6-1+ pancreatic progenitor cells.

10. The method of claim 9 wherein step a) II) further comprises a wnt signaling agonist, preferably Wnt3a CIHR 99021 or wherein step b) further comprises contacting the Stage I cells with one or more of a wnt signaling agonist, preferably Wnt3a.

11. The method of any one of claims 1 to 10 comprising enriching or isolating a NKX6-1 positive population of cells; wherein the enriching or isolating step comprises contacting the population of cells with an HPx epitope detecting antibody and isolating or partially purifying the HPx1 and/or HPx2 bound subpopulation of cells.

12. The method of claim 11, wherein the HPx epitope detecting antibody is HPx1 and/or HPx2.

## Patentansprüche

1. Verfahren zum Herstellen von NKX6-1+-Pankreas-Vorläuferzellen aus einer entodermalen Zellpopulation, wobei das Verfahren ein Kontaktieren der entodermalen Zellpopulation mit einer Kombination aus 1) mindestens einer EGF-Komponente, die ausgewählt ist aus EGF, einem aktiven Konjugat von EGF und einem aktiven Fragment von EGF, und 2) Nicotinamid oder einem Salz davon umfasst, um die Differenzierung zumindest eines Teils der entodermalen Zellpopulation zu NKX6-1+-Pankreas-Vorläuferzellen hervorzurufen.

2. Verfahren nach Anspruch 1, wobei die Kombination ferner mindestens eine Noggin-Komponente umfasst, die ausgewählt ist aus Noggin, einem aktiven Konjugat von Noggin, einem aktiven Fragment von Noggin, BMPR1A, BMPR1B, Dorsomorphin, LDN193189, Chordin und einer Kombination aus zweien oder mehreren der Genannten.

3. Verfahren nach Anspruch 1 oder 2, wobei es sich bei der EGF-Komponente um EGF oder Heparin bindenden EGF (HB-EGF) handelt.

4. Verfahren nach Anspruch 2 oder 3, wobei die Kombination Noggin, EGF und Nicotinamid umfasst.

5. Verfahren nach einem der Ansprüche 1 bis 4, wobei die Kombination ferner Exendin-4 oder ein aktives Fragment davon umfasst.

6. Verfahren nach einem der Ansprüche 1 bis 5, wobei die entodermale Zellpopulation aus pluripotenten Stammzellen (PSCs), bevorzugt einer embryonalen Stammzelle (ESC) oder einer induzierten pluripotenten Stammzelle (iPSCs), differenziert wird.

7. Verfahren nach Anspruch 6, wobei es sich bei der pluripotenten Stammzelle um eine humane ESC (hESC) oder eine humane iPSC (hiPSC) handelt.

8. Verfahren nach einem der Ansprüche 1 bis 7, umfassend ein zunächst erfolgendes Herstellen einer entodermalen Zellpopulation.

9. Verfahren nach Anspruch 8, wobei das Verfahren zum Herstellen der entodermalen Zellpopulation einen oder mehrere der Schritte oder Teilschritte a, b und c sowie Schritt d umfasst, wobei die Schritte umfassen:
a) Kontaktieren einer pluripotenten Stammzellpopulation mit einer Kombination aus:
I) einem Nodal-Agonisten, bevorzugt ActA, und einem Wnt-Signal-Agonisten, bevorzugt Wnt3a oder CHIR 99021,
II) einem Nodal-Agonisten, bevorzugt ActA, einem FGF-Agonisten, bevorzugt bFGF, und
III) einem Nodal-Agonisten, bevorzugt ActA, und einem FGF-Agonisten, bevorzugt bFGF,
um differenzierte Zellen des Stadiums 1 herzustellen,
b) Kontaktieren der differenzierten Zellen des Stadiums 1 mit einem FGF-Agonisten, bevorzugt FGF10, und einer Noggin-Komponente, bevorzugt Dorsomorphin, um differenzierte Zellen des Stadiums 2 herzustellen,
c) Kontaktieren der differenzierten Zellen des Stadiums 2 mit Noggin, einer Retinsäure (RA) oder einem RA-Analogon, einem Hedgehog-Signalhemmer, bevorzugt Cyclopamin-KAAD (Cyc), einem FGF-Agonisten, bevorzugt FGF10, und Exendin-4, um eine entodermale Zellpopulation bereitzustellen, und
d) Kontaktieren der entodermalen Zellpopulation mit einer Kombination, die mindestens eine EGF-Komponente, die ausgewählt ist aus EGF, einem aktiven Konjugat von EGF und einem aktiven Fragment von EGF, und Nicotinamid oder ein Salz davon umfasst, in einer Menge, die ausreicht, um die Differenzierung zumindest eines Teils der entodermalen Zellpopulation zu NKX6-1+-Pankreas-Vorläuferzellen hervorzurufen.

10. Verfahren nach Anspruch 9, wobei der Schritt a) II) ferner einen Wnt-Signal-Agonisten, bevorzugt Wnt3a oder CHIR 99021, umfasst oder wobei Schritt b) ferner ein Kontaktieren der Zellen des Stadiums 1 mit einem oder mehreren Wnt-Signal-Agonisten, bevorzugt Wnt3a, umfasst.

11. Verfahren nach einem der Ansprüche 1 bis 10, umfassend ein Anreichern oder Isolieren einer NKX6-1-positiv-Zellpopulation, wobei der Schritt des Anreicherns oder Isolierens ein Kontaktieren der Zellpopulation mit einem HPx-Epitop detektierenden Antikörper und Isolieren oder teilweises Reinigen der HPx1- und/oder HPx2-gebundenen Teilpopulation umfasst.

12. Verfahren nach Anspruch 11, wobei es sich bei dem HPx-Epitop detektierenden Antikörper um HPx1 und/oder HPx2 handelt.

## Revendications

1. Procédé de production de cellules progénitrices pancréatiques NKX6-1+ à partir d'une population de cellules endodermiques, le procédé comprenant la mise en contact de la population de cellules endodermiques avec une combinaison de 1) au moins un composant EGF choisi parmi l'EGF, un conjugué actif d'EGF et un fragment actif d'EGF ; et 2) nicotinamide ou un sel de celui-ci pour induire la différenciation d'au moins une partie de la population de cellules endodermiques en cellules progénitrices pancréatiques NKX6-1+.

2. Procédé selon la revendication 1, dans lequel la combinaison comprend en outre au moins un composant noggine choisi parmi la noggine, un conjugué actif de noggine, un fragment actif de noggine, le BMPR1A, le BMPR1B, la dorsomorphine, le LDN193189, la chordine et toute combinaison de deux de ces composants ou plus.

3. Procédé selon la revendication 1 ou 2, dans lequel le composant EGF est l'EGF ou l'EGF se liant à l'héparine (HB-EGF).

4. Procédé selon la revendication 2 ou 3, dans lequel la combinaison comprend de la noggine, de l'EGF et du nicotamide.

5. Procédé selon l'une quelconque des revendications 1 à 4, dans lequel la combinaison comprend en outre de l'extendine-4 ou un fragment actif de celle-ci.

6. Procédé selon l'une quelconque des revendications 1 à 5, dans lequel la population de cellules endodermiques est différenciée de cellules souches pluripotentes (CSP), de préférence une cellule souche embryonnaire (CSE) ou une cellule souche pluripotente induite (CSPi)

7. Procédé selon la revendication 6, dans lequel la cellule souche pluripotente est une CSE humaine (CSEh) ou une CSPi humaine (CSPih).

8. Procédé selon l'une quelconque des revendications 1 à 7, comprenant tout d'abord la production d'une population de cellules endodermiques.

9. Procédé selon la revendication 8, ledit procédé de production de la population de cellules endodermiques comprenant une ou plusieurs des étapes ou sous-étapes a, b et c ; et l'étape d, les étapes comprenant :
a) la mise en contact d'une population de cellules souches pluripotentes avec une combinaison de :
I) un agoniste nodal, de préférence l'ActA et un agoniste de la signalisation wnt, de préférence le Wnt3a ou le CHIR 99021,
II) un agoniste nodal, de préférence l'ActA, un agoniste de FGF, de préférence le bFGF, et
III) un agoniste nodal, de préférence l'ActA, et un agoniste de FGF, de préférence le bFGF ;
pour produire des cellules différenciées au stade 1 ;
b) la mise en contact des cellules différenciées au stade 1 avec un agoniste de FGF, de préférence le FGF10, et un composant noggine, de préférence la dorsomorphine, pour produire des cellules différenciées au stade 2 ;
c) la mise en contact des cellules différenciées au stade 2 avec de la noggine, de l'acide rétinoïque (AR) ou un analogue d'AR, un inhibiteur de la signalisation hedgehog, de préférence la cyclopamine-KAAD (Cyc), un agoniste de FGF, de préférence le FGF10, et l'extendine-4 pour fournir une population de cellules endodermiques ; et
d) la mise en contact de la population de cellules endodermiques avec une combinaison comprenant au moins un composant EGF choisi parmi l'EGF, un conjugué actif d'EGF et un fragment actif d'EGF, et du nicotamide ou un sel de celui-ci en quantité suffisante pour induire la différenciation d'au moins une partie de la population de cellules endodermiques en cellules progénitrices pancréatiques NKX6-1+.

10. Procédé selon la revendication 9 dans lequel l'étape a) II) comprend en outre un agoniste de la signalisation wnt, de préférence le Wnt3a CIHR 99021 ou dans lequel l'étape b) comprend en outre la mise en contact des cellules au stade I avec un ou plusieurs agonistes de la signalisation wnt, de préférence le Wnt3a.

11. Procédé selon l'une quelconque des revendications 1 à 10 comprenant l'enrichissement ou l'isolement d'une population de cellules NKX6-1 positive ; ladite étape d'enrichissement ou d'isolement comprenant la mise en contact de la population de cellules avec un anticorps de détection d'épitope HPx et l'isolement ou la purification partielle de la sous-population de cellules liée à HPx1 et/ou HPx2.

12. Procédé selon la revendication 11, dans lequel l'anticorps de détection d'épitope HPx est HPx1 et/ou HPx2.
